# EUROPÄISCHE PATENTSCHRIFT

(11) **EP 1 017 674 B1**
(45) Veröffentlichungstag und Bekanntmachung des Hinweises auf die Patenterteilung: **18.02.2009**
(21) Anmeldenummer: 98951386.6
(22) Anmeldetag: 12.09.1998
(51) Int. Cl.: C07D 209/54, C07D 307/94, C07C 233/52, C07C 69/614, A01N 43/12, A01N 43/38

(54) **SPIROCYCLISCHE PHENYLKETOENOLE ALS PESTIZIDE UND HERBIZIDE**
UTILIZING SPIROCYCLIC PHENYL KETO-ENOLS AS PESTICIDES AND HERBICIDES
UTILISATION D'ENOLS CETONIQUES DE PHENYLE SPIROCYCLIQUES COMME PESTICIDES ET HERBICIDES

(30) Priorität: 26.09.1997 DE 19742492
(43) Veröffentlichungstag der Anmeldung: 12.07.2000
(73) Patentinhaber: Bayer CropScience AG, 40789 Monheim (DE)
(72) Erfinder: FISCHER, Reiner, D-40789 Monheim (DE); BRETSCHNEIDER, Thomas, D-53797 Lohmar (DE); ERDELEN, Christoph, D-42799 Leichlingen (DE); WACHENDORFF-NEUMANN, Ulrike, D-56566 Neuwied (DE); DOLLINGER, Markus, Overland Park, KS 66213 (US); TURBERG, Andreas, D-42781 Haan (DE)
(86) Internationale Anmeldenummer: PCT/EP1998/005809
(87) Internationale Veröffentlichungsnummer: WO 1999/016748

(56) Entgegenhaltungen:
- EP-A- 0 596 298
- WO-A-95/26954
- WO-A-96/35664
- DE-A- 4 337 853
- DE-A- 4 431 730
- DE-A- 19 602 524
- DE-A- 19 603 332

## Beschreibung

Die Erfindung betrifft neue spirocyclische Phenylketoenole, mehrere Verfahren und Zwischenprodukte zu ihrer Herstellung und ihre Verwendung als Schädlingsbekämpfungsmittel und Herbizide.

Es ist bereits bekannt geworden, daß bestimmte phenylsubstituierte cyclische Ketoenole als Insektizide, Akarizide bzw. Herbizide wirksam sind, beispielsweise 1H-Arylpyrrolidin-dion-Derivate (EP-A-456 063, EP-A-521 334, EP-A-596 298, EP-A-613 884, EP-A-613 885, DE-44 40 594, WO 94/01 997, WO 95/01 358, WO 95/26 954, WO 95/20 572, EP-A-0 668 267, WO 96/25 395, WO 96/35 664, WO 97/01 53 5 und WO 97/02 243).

Es ist ferner bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)- Δ³-dihydrofuraatin-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl- Δ³-dihydrofuranon-Derivate mit herbiziden, akariziden und insektiziden Eigenschaften aus EP-A-528 156, EP-A 0 647 637, WO 95/26345, WO 96/20 196, WO 96/25 395, WO 96/35 664, WO 97/01 535, WO 97/02 243 und WO 97/36 868 bekannt.

Die Wirksamkeit und Wirkungsbreite dieser Verbindungen ist jedoch insbesondere bei niedrigen Aufwandmengen und Konzentrationen nicht immer ganz zufriedenstellend. Weiterhin ist die Pflanzenverträglichkeit dieser Verbindungen nicht immer ausreichend.

Es wurden nun neue Verbindungen der Formel (I) 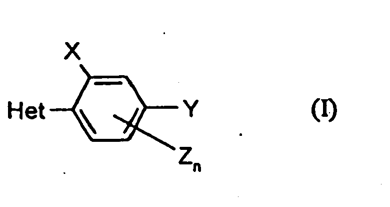 gefunden,
in welcher
- X: für Chlor, Methyl, steht,
- Y: für Wasserstoff, Chlor, Methyl, steht,
- Z: für Chlor, Methyl,
- n: für 0, 1 oder 2 steht,
- Het: für eine der Gruppen 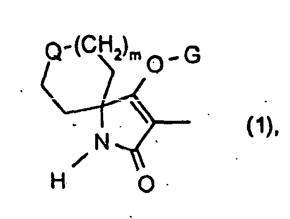 steht,
- G: für Wasserstoff (a) oder für eine der Gruppen 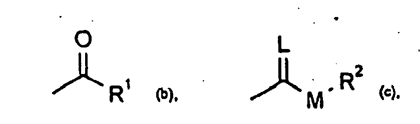 steht,
worin
L für Sauerstoff steht und
M für Sauerstoff steht,
- R¹: für jeweils gegebenenfalls durch Fluor oder Chlor substituiertes C₁-C₁₄-Alkyl, C₂-C₁₄-Alkenyl, C₁-C₄-Alkoxy-C₁-C₄-alkyl oder C₁-C₄-Alkylthio-C₁-C₆-alkyl oder für gegebenenfalls durch Methyl, Ethyl, tert.-Butyl oder Methoxy substituiertes C₃-C₆-Cycloalkyl,
für gegebenenfalls durch Fluor, Chlor, Brom, Cyano, Nitro, Methyl, Methoxy, Trifluormethyl oder Trifluormethoxy substituiertes Phenyl,
für gegebenenfalls durch Fluor, Chlor, Brom, Methyl, Methoxy, Trifluormethyl oder Trifluormethoxy substituiertes Benzyl oder
für jeweils gegebenenfalls durch Fluor, Chlor, Brom oder Methyl substituiertes Furanyl, Thienyl oder Pyridyl steht,
- R²: für C₁-C₄-Alkyl, C₂-C₁₄-Alkenyl oder C₁-C₄-Alkoxy-C₂-C₆-alkyl,
für gegebenenfalls durch Methyl oder Methoxy substituiertes C₃-C₆-Cycloalkyl oder
für jeweils gegebenenfalls durch Fluor, Chlor, Brom, Cyano, Nitro, Methyl, Methoxy, Trifluormethyl oder Trifluormethoxy substituiertes Phenyl oder Benzyl steht,
- Q: für 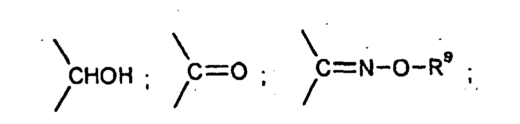 oder 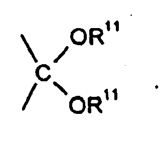 steht,
- R⁹: für Wasserstoff, für C₁-C₄-Alkyl, für C₃-C₆-Cycloalkyl oder für jeweils gegebenenfalls durch Fluor, Chlor, Brom, Methyl, Ethyl, n-Propyl, iso-Propyl, tert.-Butyl, Methoxy, Trifluormethyl, Trifluormethoxy, Nitro oder Cyano substituiertes Phenyl oder Benzyl der für CO-R^{1'}, CO₂R^{2'}, SO₂R^{1'}, CONH₂, CONHR¹¹ oder 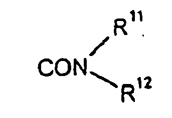 steht,
- R¹¹ und R¹²: gleich oder verschieden sind und für Methyl oder Ethyl steht,
- m: für 1 steht,
- R^{1'}: unabhängig von R¹ für die oben für R¹ als ganz besonders bevorzugt genannten Bedeutungen steht und
- R^{2'}: unabhängig von R² für die oben für R² als ganz besonders bevorzugt genannten Bedeutungen steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutung (1) der Gruppe Het ergibt sich folgende hauptsächliche Struktur (I-1): 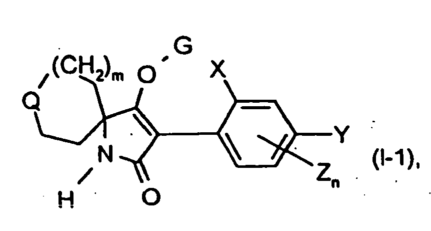 worin
Q, G, X, Y, Z, n und m die oben angegebenen Bedeutungen haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn Het für die Gruppe (1) steht, 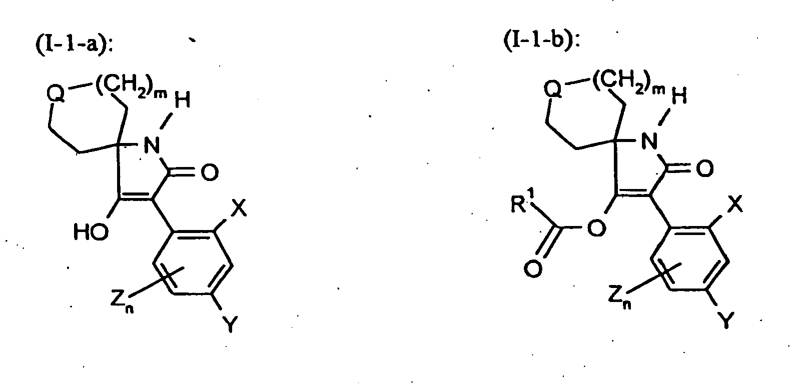 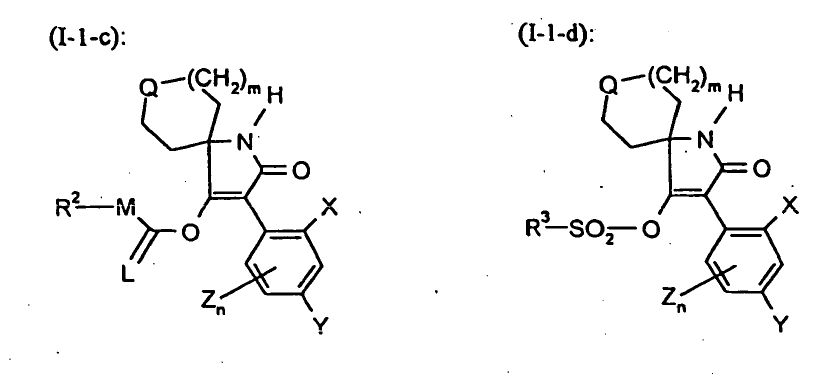 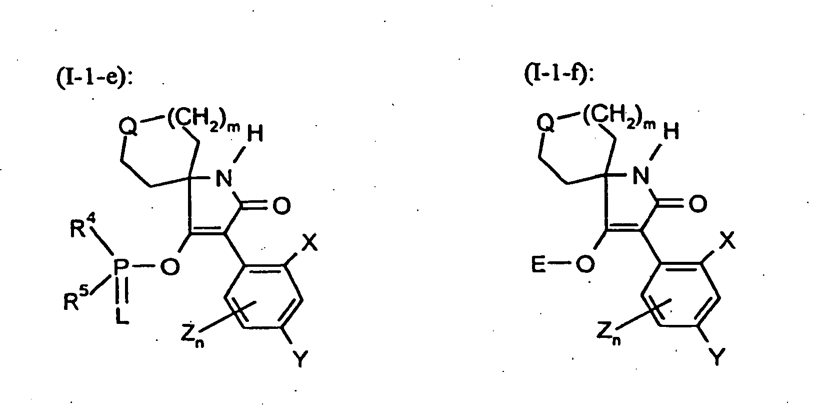 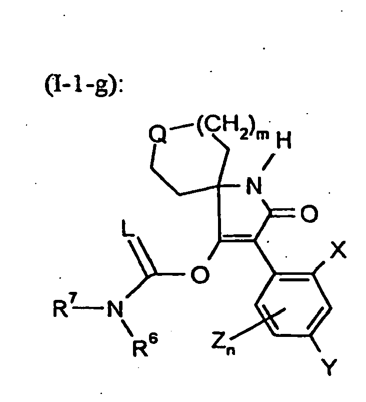 worin
E, L, M, Q, X, Y, Z, n, m, R¹, R², R³, R⁴, R⁵, R⁶ und R⁷ die oben angegebenen Bedeutungen besitzen.

Weiterhin wurde gefunden, daß man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 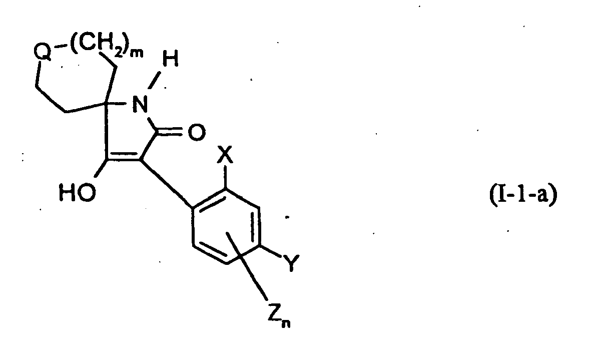 in welcher
   Q, X, Y, Z, m und n die oben angegebenen Bedeutungen haben,
   wenn man
   N-Acylaminosäureester der Formel (II) 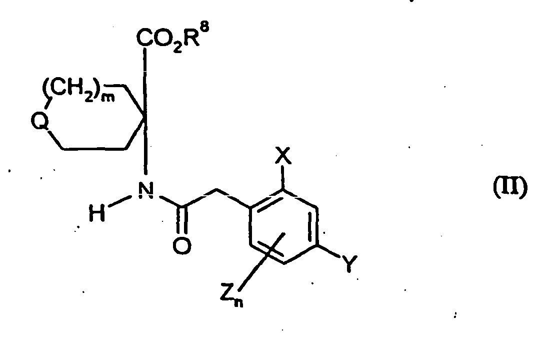 in welcher
   Q, X, Y, Z, m und n die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
   Außerdem wurde gefunden, daß man die Verbindungen der oben gezeigten Formel (I-1-b), in welchen
(D) Q, X, Y, Z, m, n und R¹ die oben angegebenen Bedeutungen haben, erhält, wenn man Verbindungen der oben gezeigten Formel (I-1-a), in welchen Q, X, Y, Z, m und n die oben angegebenen Bedeutungen haben, jeweils
   α) mit Säurechloriden der Formel (V) 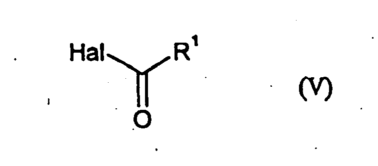 in welcher
      - R¹: die oben angegebenen Bedeutungen hat und
      - Hal: für Halogen (insbesondere Chlor oder Brom) steht
      oder
   β) mit Carbonsäureanhydriden der Formel (VI)

      R¹-CO-O-CO-R¹ (VI)

      in welcher
      - R¹: die oben angegebenen Bedeutungen hat,
      gegebenenfalls in Gegenwart eines Verdünnungsmittels und gegebenenfalls in Gegenwart eines Säurebindemittels umsetzt;
(E) daß man die Verbindungen der oben gezeigten Formel (I-1-c) in welchen Q, R², M, X, Y, Z, m und n die oben angegebenen Bedeutungen haben und L für Sauerstoff steht, erhält, wenn man Verbindungen der oben gezeigten Formel (I-1-a), in welchen Q, X, Y, Z, m und n die oben angegebenen Bedeutungen haben, jeweils
   mit Chlorameisensäureestern oder Chlorameisensäurethiolestern der Formel (VII)

   R²-M-CO-Cl (VII)

   in welcher
   - R² und M: die oben angegebenen Bedeutungen haben,
   gegebenenfalls in Gegenwart eines Verdünnuugsmittels und gegebenenfalls in Gegenwart eines Säurebindemittels umsetzt;
(F) daß man Verbindungen der oben gezeigten Formel (I-1-c), in welchen Q, R², M, X, Y, Z, m und n die oben angegebenen Bedeutungen haben und L für Schwefel steht, erhält, wenn man Verbindungen der oben gezeigten Formel (I-1-a), in welchen Q, X, Y, Z, m und n die oben angegebenen Bedeutungen haben, jeweils
   mit Chlormonothioameisensäureestern oder chlordithioameisensäureestern der Formel (VIII) 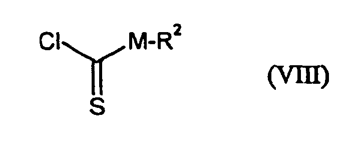 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt,
(G) daß man Verbindungen der oben gezeigten Formel (I-1-d), in welchen Q, R³, X, Y, Z, m und n die oben angegebenen Bedeutungen haben, erhält, wenn man Verbindungen der oben gezeigten Formel (I-1-a), in welchen Q, X, Y, Z, m und n die oben angegebenen Bedeutungen haben, jeweils
   mit Sulfonsäurechloriden der Formel (IX)

   R³-SO₂-Cl (IX)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(H) daß man Verbindungen der oben gezeigten Formel (I-1-e), in welchen Q, L, R⁴, R⁵, X, Y, Z, m und n die oben angegebenen Bedeutungen haben, erhält, wenn man Verbindungen der oben gezeigten Formel (I-1-a), in welchen Q, X, Y, Z, m und n die oben angegebenen Bedeutungen haben, jeweils
   mit Phosphorverbindungen der Formel (X) 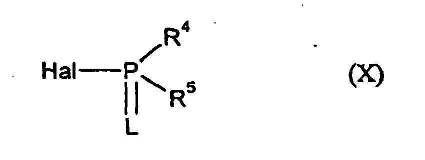 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(I) daß man Verbindungen der oben gezeigten Formel (I-1-f), in welchen Q, E, X, Y, Z, m und n die oben angegebenen Bedeutungen haben, erhält, wenn man Verbindungen der oben gezeigten Formel (I-1-a), in welchen Q, X, Y, Z, m und n die oben angegebenen Bedeutungen haben, jeweils
   mit Metallverbindungen oder Aminen der Formeln (XI) oder (XII)

   Me(OR¹³)ₜ (XI)

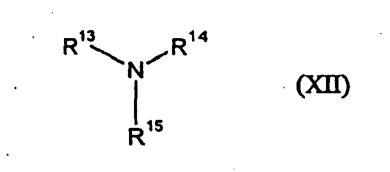

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für 1 oder 2 und
   - R¹³, R¹⁴, R¹⁵: unabhängig voneinander für Wasserstoff oder Alkyl, (bevorzugt C₁-C₈-Alkyl) stehen,
   gegebenenfalls in Gegenwart eines Verdünnungsmittels umsetzt,
(J) daß man Verbindungen der oben gezeigten Formel (I-1-g) in welchen Q, L, R⁶, R⁷, X, Y, Z, m und n die oben angegebenen Bedeutungen haben, erhält, wenn man Verbindungen der oben gezeigten Formel (I-1-a), in welchen Q, Y, Y, Z, m und n die oben angegebenen Bedeutungen haben, jeweils
   α) mit Isocyanaten oder Isothiocyanaten der Formel (XIII)

      R⁶-N=C=L (XIII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XIV) 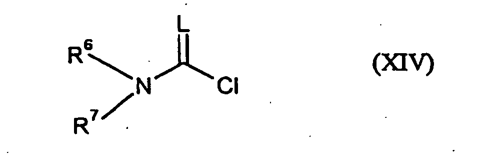 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt.

Die erfindungsgemäßen Verbindungen der Formel (I-1-a), sind somit wichtige Zwischenprodukte für die Herstellung der erfindungsgemäßen Verbindungen der Formel (I-1), in welchen G jeweils für eine der Gruppen b), c), d), e), f) oder g) steht.

Weiterhin wurde gefunden, daß die neuen Verbindungen der Formel (I) eine sehr gute Wirksamkeit als Schädlingsbekämpfungsmittel, vorzugsweise als Insektizide und Akarizide und als Herbizide aufweisen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

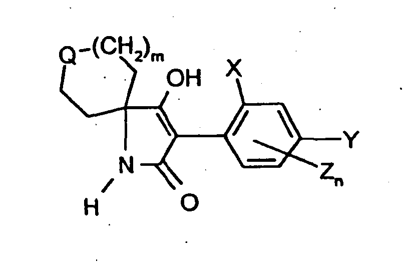

**Tabelle 1:**

| | | | | | |
|---|---|---|---|---|---|
| m = 1 | | | | | |
| 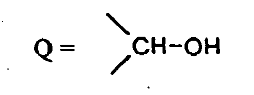 | | | | | |

| **X** | **Y** | **Zₙ** | **X** | **Y** | **Zₙ** |
|---|---|---|---|---|---|
| CH₃ | H | H | CH₃ | Br | 6-Cl |
| CH₃ | CH₃ | H | CH₃ | Cl | 6-Br |
| CH₃ | H | 5- CH₃ | Br | CH₃ | 6-Cl |
| CH₃ | H | 6- CH₃ | CH₃ | CH₃ | 6-CN |
| CH₃ | CH₃ | 5- CH₃ | CH₃ | CN | 6-CH₃ |
| CH₃ | CH₃ | 6- CH₃ | CH₃ | CH₃ | 6-OCH₃ |
| CH₃ | CH₃ | 3,5-( CH₃)₂ | CH₃ | OCH₃ | 6-CH₃ |
| CH₃ | CH₃ | 3,6-(CH₃)₂ | Cl | Cl | 5-Cl |
| CH₃ | Cl | H | CH₃ | Cl | 5-CH₃ |
| Cl | CH₃ | H | Br | CH₃ | 5-Cl |
| Cl | H | 6-F | Br | CH₃ | 5-CH₃ |
| CH₃ | H | 6-Cl | CH₃ | Br | 5-Cl |
| Cl | H | 6-Cl | Cl | Cl | 5-CH₃ |
| CH₃ | Cl | 6-Cl | CH₃ | Br | 5-CH₃ |
| CH₃ | Br | 5-Br | Cl | CH₃ | 5-Cl |
| Cl | CH₃ | 6-Cl | CH₃ | CH₃ | 5-Br |
| Br | CH₃ | 6-Br | CH₃ | H | 3-Cl, 6-CH₃ |
| CH₃ | Cl | 6-CH₃ | CH₃ | H | 3-Br, 6-CH₃ |
| CH₃ | CH₃ | 6-Cl | Cl | CF₃ | 6-Cl |
| CH₃ | Br | 6-CH₃ | | | |
| CH₃ | CH₃ | 6-Br | | | |

### Tabellen 2 bis 8

### Verbindungen der Formel (I-1-a)

- **Tabelle 2:**: X, Y und Zₙ wie in Tabelle 1 angegeben
m = 1 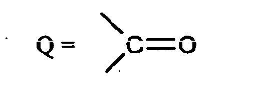
- **Tabelle 4:**: X, Y und Zₙ wie in Tabelle 1 angegeben
m = 1

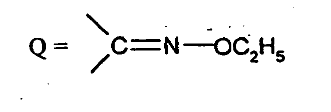

Verwendet man gemäß Verfahren (A) N-[(2,4,6-Trimethyl-phenyl)-acetyl]-1-amino-4-methoximino-cyclohexan-carbonsäuremethylester als Ausgangsstoff, so kann der Verlauf des erfindungsgemäßen Verfahrens durch folgendes Reaktionsschema wiedergegeben werden:

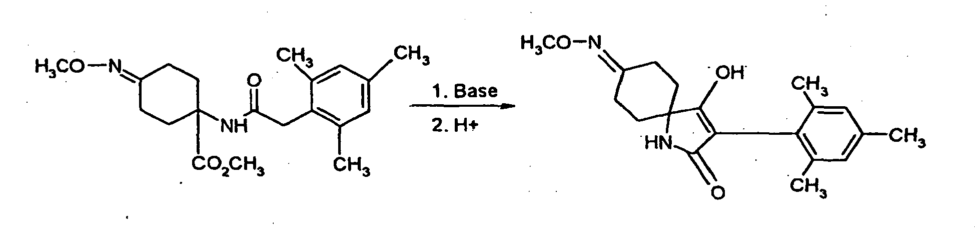

Verwendet man gemäß Verfahren (Dα) 3-[(2-Chlor-4-methyl)-phenyl]-5,5-[(3-phenylimino)-pentamethylen]-pyrrolidin-2,4-dion und Pivaloylchlorid als Ausgangsstoffe, so kann der Verlauf des erfindungsgemäßen Verfahrens durch folgendes Reaktionsschema wiedergegeben werden:

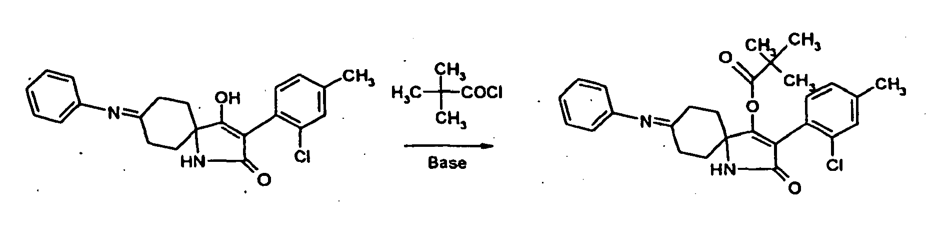

Verwendet man gemäß Verfahren (E) 3-[(2,4-Dichlor-6-methyl)-phenyl]-5,5-[(3-Oxo-pentamethylen)]-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

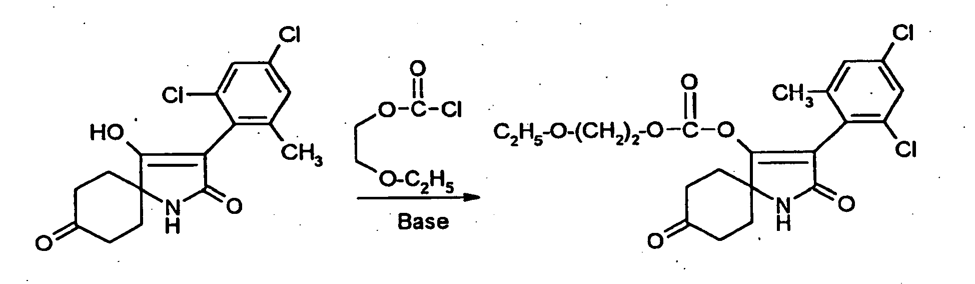

Verwendet man gemäß Verfahren (H) 3-[(2,5-Dichlor)-phenyl]-5,5-[(3-oxa)-pentamethylen]-pyrrolidin-2,4-dion und Methanthio-phosphonsäurechlorid-(2,2,2-trifluorethylester) als Ausgangsstoffe, so kann der Reaktionsverlauf durch folgendes Reaktionsschema wiedergegeben werden:

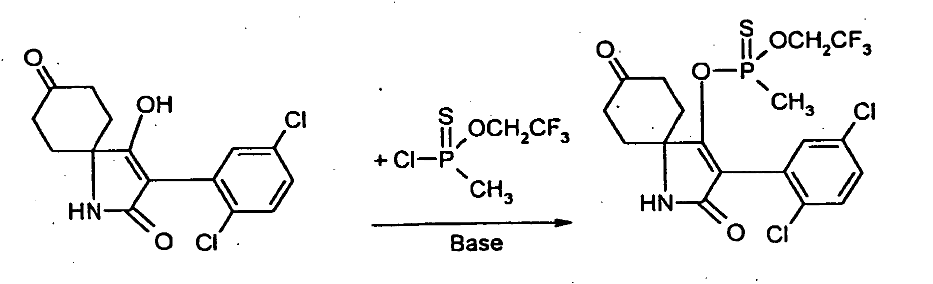

Verwendet man gemäß Verfahren (I) 3-[(2,4-Dichlor)-phenyl]-5,5-[(3-ethoximino)-pentamethylen]-pyrrolidin-2,4-dion und NaOH als Ausgangsstoffe, so kann der Verlauf des erfindungsgemäßen Verfahrens durch folgendes Reaktionsschema wiedergegeben werden:

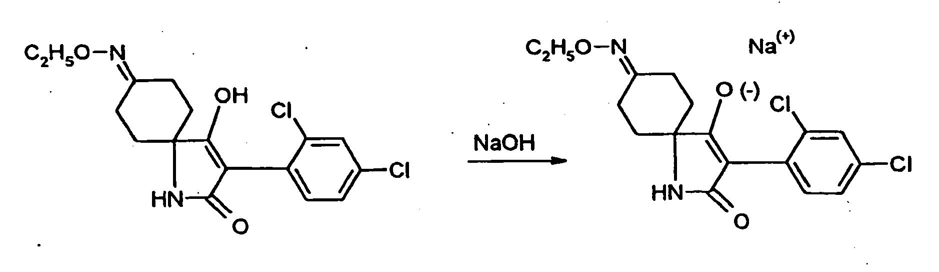

Verwendet man gemäß Verfahren (Jβ) 3-[2,3,4,6-Tetramethyl-phenyl]-5,5-[(4-methoximino)-pentamethylen]-pyrrolidin-2,4-dion und Dimethylcarbamidsäurechlorid als Ausgangsstoffe, so kann der Reaktionsverlauf durch folgendes Schema wiedergegeben werden:

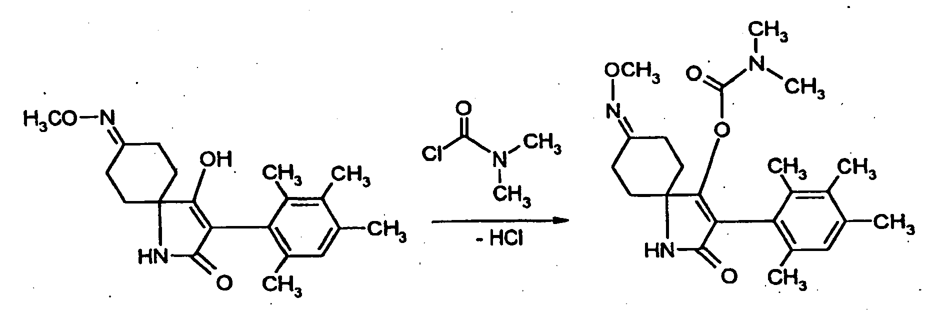

Die beim erfindungsgemäßen Verfahren (A) als Ausgangsstoffe benötigten Verbindungen der Formel (II) 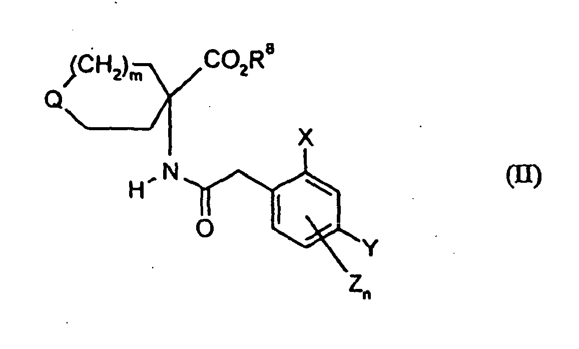 in welcher
- Q, X, Y, Z, m, n und R⁸: die oben angegebenen Bedeutungen haben,
sind neu.

Man erhält die Acylaminosäureester der Formel (II) beispielsweise, wenn man Aminosäurederivate der Formel (XV) 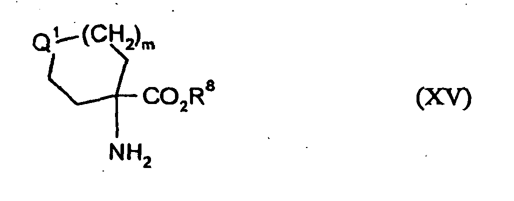 in welcher
- Q¹, m und R⁸: die oben angegebenen Bedeutungen haben,
mit substituierten Phenylessigsäurehalogeniden der Formel (XVI) 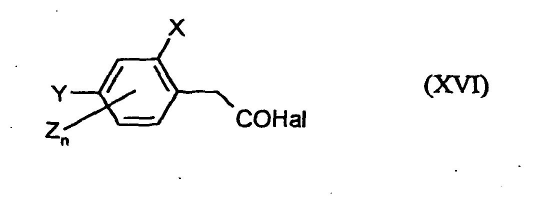 in welcher
- X, Y, Z und n: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968),
oder wenn man Acylaminosäuren der Formel (XVII) 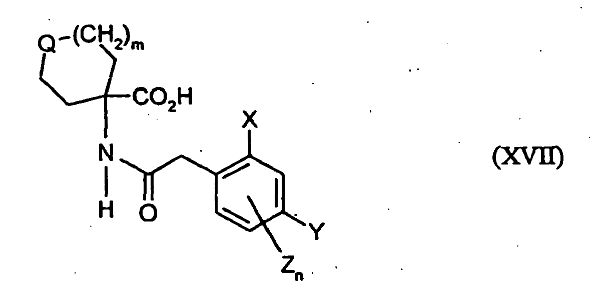 in welcher
- Q, m, X, Y, Z und n: die oben angegebenen Bedeutungen haben,
verestert (Chem. Ind. (London) 1568 (1968)).

Die Verbindungen der Formel (XVII) 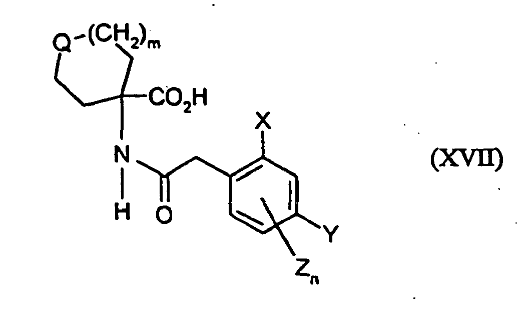 in welcher
- Q, m, X, Y, Z und n: die oben angegebenen Bedeutungen haben,
sind neu.

Man erhält Verbindungen der Formel (XVII), wenn man Aminosäuren der Formel (XVIII) 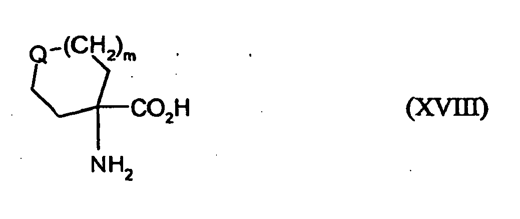 in welcher
- Q und m: die oben angegebenen Bedeutungen haben,
mit substituierten Phenylessigsäurehalogeniden der Formel (XVI) 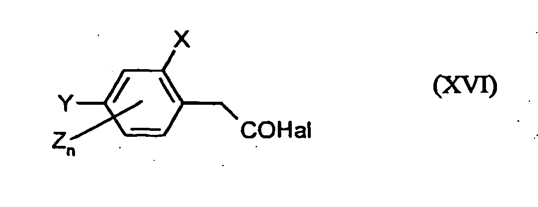 in welcher
- X, Y, Z und n: die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XVI) sind teilweise bekannt. Sie lassen sich nach bekannten Verfahren darstellen (s. z.B. Henecka, Houben-Weyl, Methoden der Organischen Chemie, Bd. 8, S. 467-469 (1952)) oder sind bekannt aus den eingangs zitierten Patentanmeldungen bzw. WO 98/05638 und WO 97/36868.

Weiterhin lassen sich die bei dem obigen Verfahren (A) verwendeten Ausgangsstoffe der Formel (II) 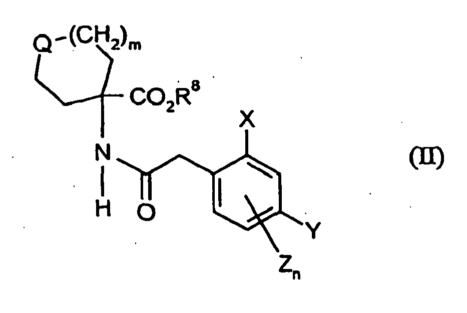 in welcher
- Q, X, Y, Z, m, n und R⁸: die oben angegebenen Bedeutungen haben,
herstellen, wenn man Aminonitrile der Formel (XIX) 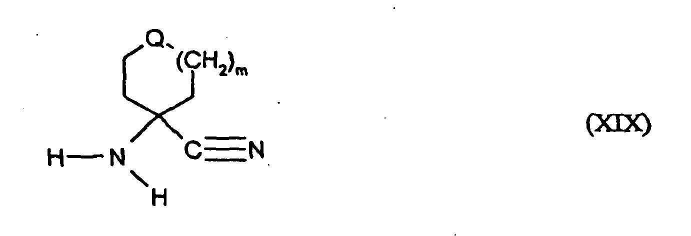 in welcher
- Q und m: die oben angegebenen Bedeutungen haben,
mit substituierten Phenylessigsäurehalogeniden der Formel 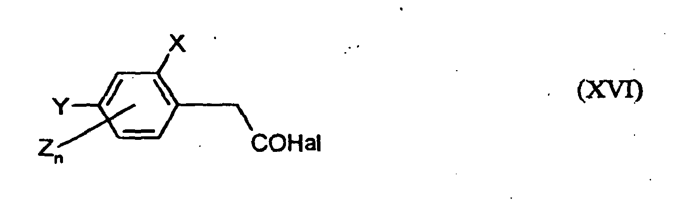 in welcher
- X, Y, Z, n und Hal: die oben angegebenen Bedeutungen haben,
zu Verbindungen der Formel (XX) 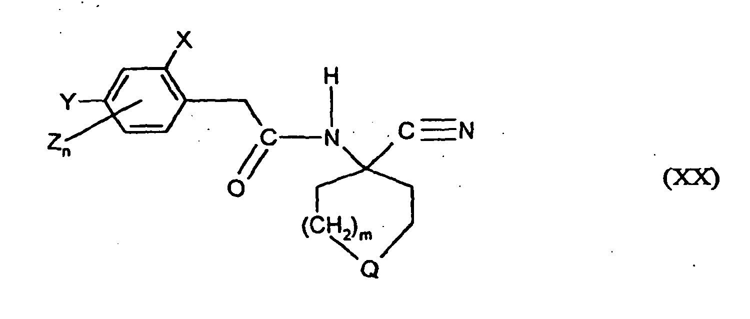 in welcher
- Q, X, Y, Z, m und n: die oben angegebenen Bedeutungen haben,
umsetzt,
und diese anschließend einer sauren Alkoholyse unterwirft.

Die Verbindungen der Formel (XX) sind ebenfalls neu.

Die folgenden Reaktionsschemata zeigen beispielhaft Synthesewege zu Vorprodukten der Formel (II).

Die Großbuchstaben hinter einigen der Formelnummern dienen zur Unterscheidung unterschiedlicher Bedeutungen von Q. Sie werden zum Teil auch bei den Herstellbeispielen verwendet.

In den folgenden Reaktionsschemata wird Bezug genommen auf Verbindungen der Formeln 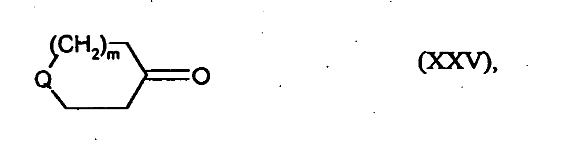 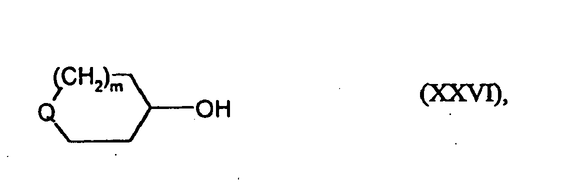 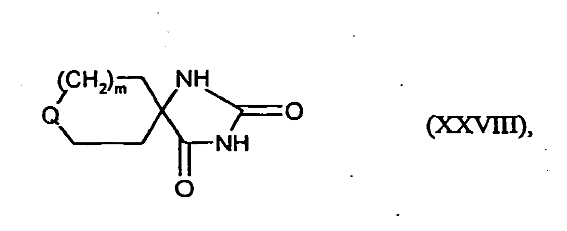 in welchen Q und m die oben angegebenen Bedeutungen haben.

In den Formelschemata sind beispielhaft Verbindungen aufgeführt für verschiedene Bedeutungen von Q und für m = 1, die aber von den oben angegebenen Formeln umfaßt werden.

Die Reste R¹¹ in 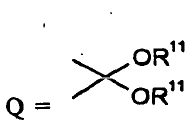 können auch gemeinsam für eine gegebenenfalls durch Methyl oder Ethyl substituierte C₂-C₃-Alkandiylgruppe stehen.

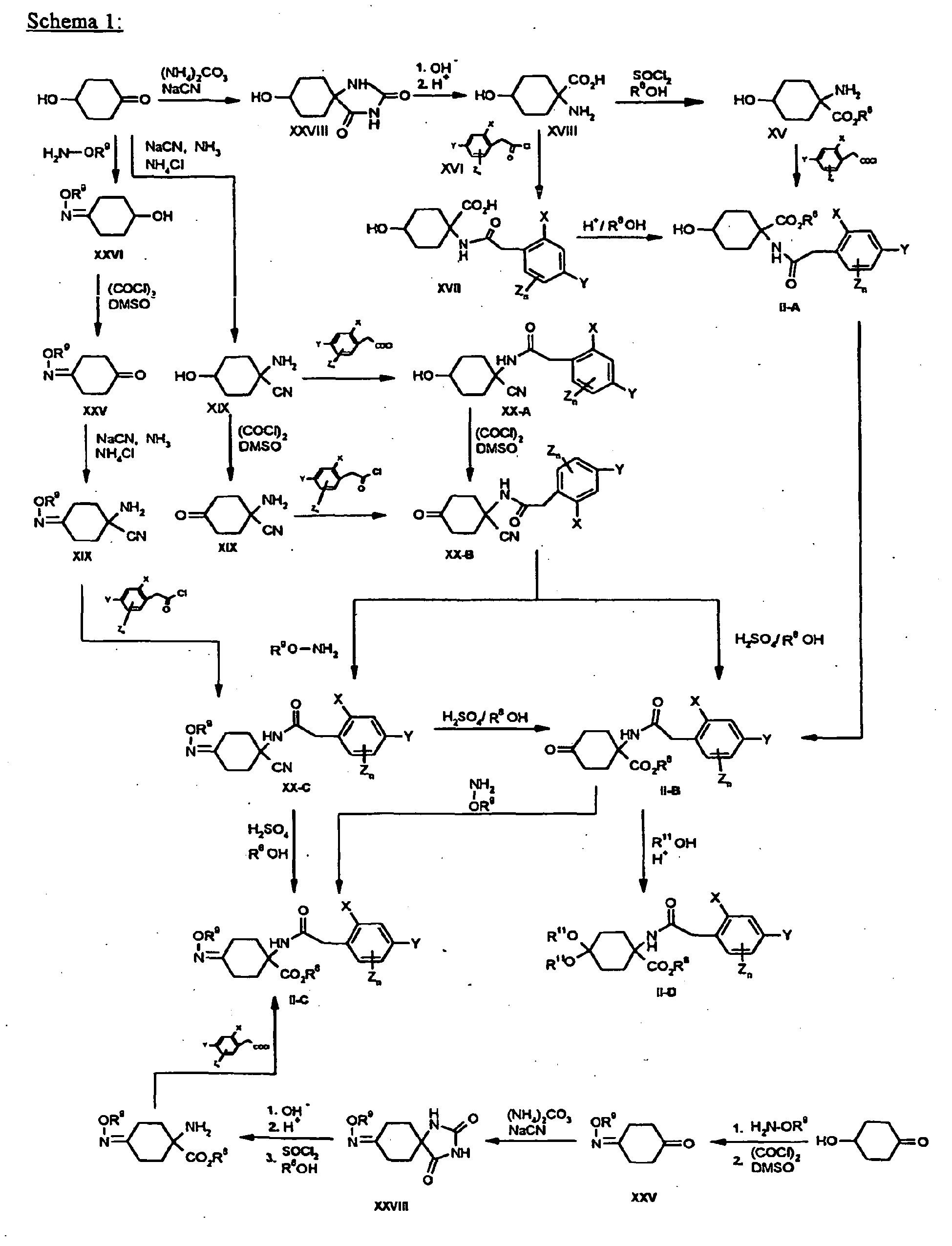

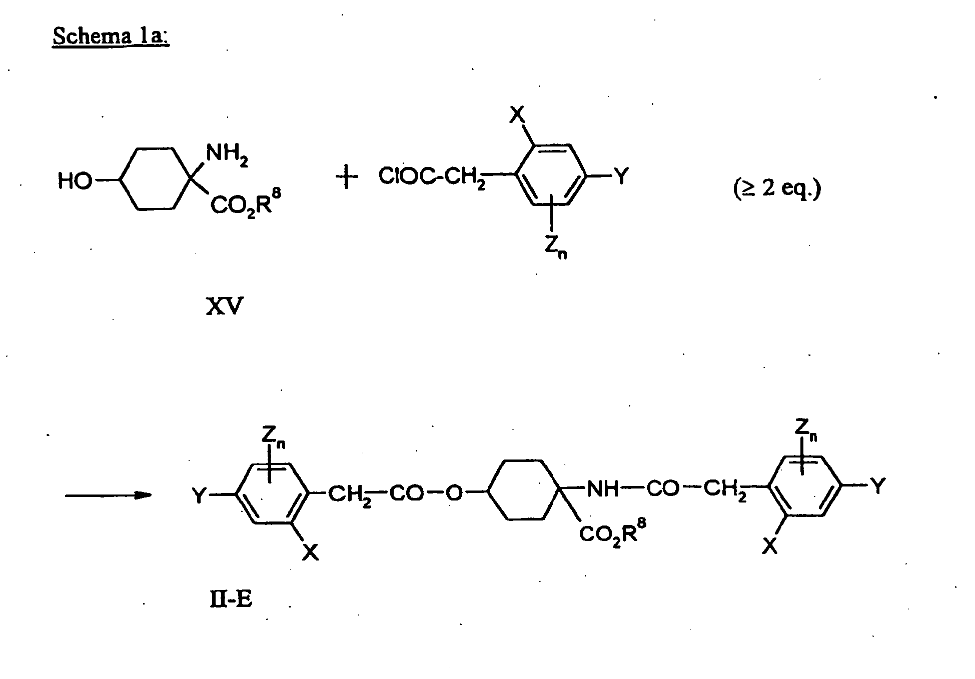

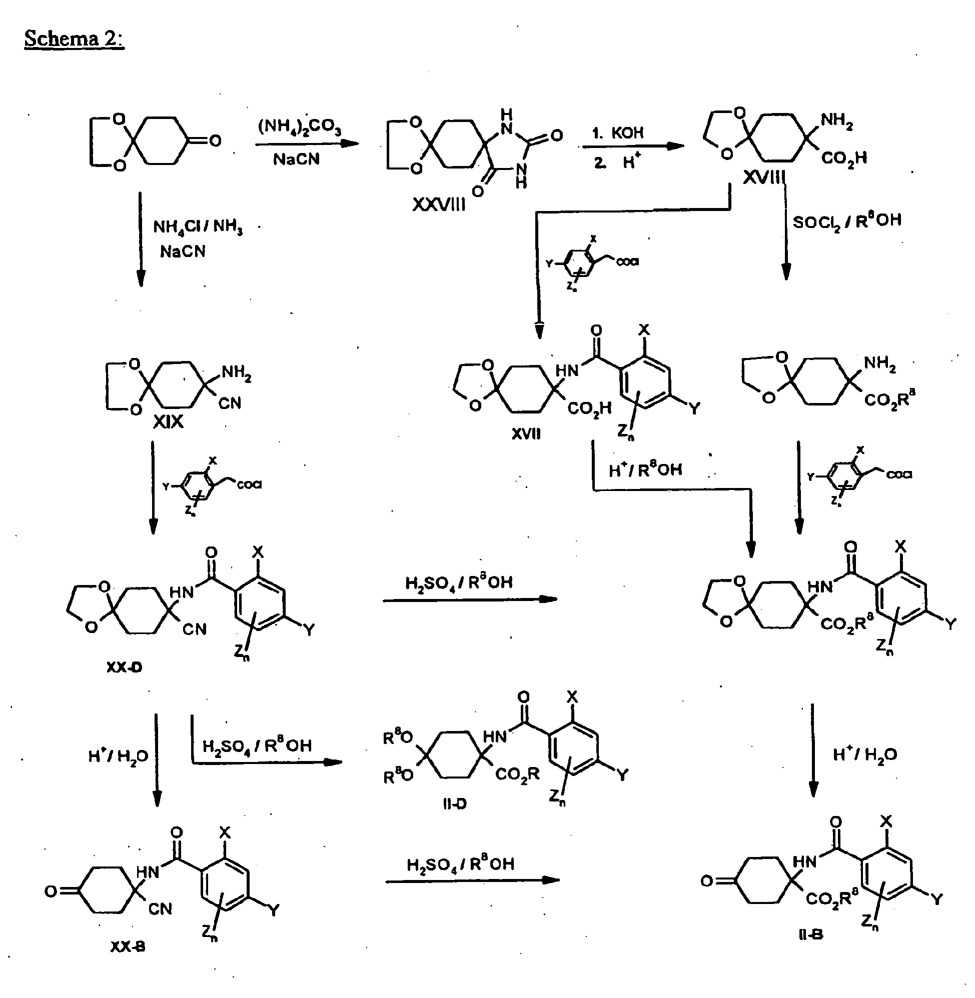

Die zur Durchführung der erfindungsgemäßen Verfahren D, E, F, G, H, I und J außerdem als Ausgangsstoffe benötigten Säurehalogenide der Formel (V), Carbonsäureanhydride der Formel (VI), Chlorameisensäureester oder Chlorameisensäurethioester der Formel (VII), Chlormonothioameisensäureester oder Chlordithioameisensäureester der Formel (VIII), Sulfonsäurechloride der Formel (IX), Phosphorverbindungen der Formel (X) und Metallhydroxide, Metallalkoxide oder Amine der Formel (XI) und (XII), Isocyanate der Formel (XIII) und Carbamidsäurechloride der Formel (XIV) sind allgemein bekannte Verbindungen der organischen bzw. anorganischen Chemie.

Die Verbindungen der Formeln (V) bis (XIV) sind darüber hinaus aus den eingangs zitierten Patentanmeldungen bekannt oder lassen sich nach den dort angegebenen Methoden herstellen.

Das Verfahren (A) ist dadurch gekennzeichnet, daß man Verbindungen der Formel (II), in welcher Q, X, Y, Z, m, n und R⁸ die oben angegebenen Bedeutungen haben, in Gegenwart einer Base einer intramolekularen Kondensation unterwirft.

Im Verfahren gemäß (A) kann in der Formel (II) Q auch für die Gruppe 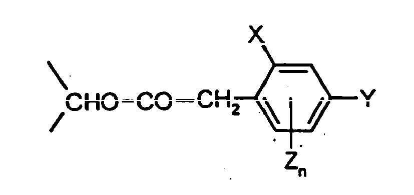 stehen, worin X, Y, Z und n die oben angegebenen Bedeutungen haben. Das gilt auch für die Bedeutung von Q in der Formel (III) im Verfahren gemäß (B).

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z:B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa äquimolaren bis doppeltäquimolaren Mengen ein. Es ist jedoch auch möglich, die eine oder andere Komponente in einem größeren Überschuß (bis zu 3 Mol) zu verwenden.

Das Verfahren (Dα) ist dadurch gekennzeichnet, daß man Verbindungen der Formel (I-1-a) jeweils mit Carbonsäurehalogeniden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Dα) alle gegenüber den Säurehalogeniden inerten Solventien eingesetzt werden. Vorzugsweise verwendbar sind Kohlenwasserstoffe, wie Benzin, Benzol Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, Nitrile wie Acetonitril, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Dα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethylanilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (Dα) innerhalb eines größeren Bereiches variiert werden. Im allgemeinen arbeitet man bei Temperaturen zwischen -20°C und +1 50°C, vorzugsweise zwischen 0°C und 100°C.

Bei der Durchführung des erfindungsgemäßen Verfahrens (Dα) werden die Ausgangsstoffe der Formel (I-1-a) und das Carbonsäurehalogenid der Formel (V) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Dβ) ist dadurch gekennzeichnet, daß man Verbindungen der Formel (I-1-a) mit Carbonsäureanhydriden der Formel (VI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Dβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Dβ) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (Dβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Dβ) werden die Ausgangsstoffe der Formel (I-1-a) und das Carbonsäureanhydrid der Formel (VI) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (E) ist dadurch gekennzeichnet, daß man Verbindungen der Formel (I-1-a) jeweils mit Chlorameisensäureestern oder Chlorameisensäurethiolestern der Formel (VII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (E) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (E) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, Nitrile wie Acetonitril, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (E) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (E) wird im allgemeinen unter Normaldruck durchgeführt.

Bei der Durchführung des erfindungsgemäßen Verfahrens (E) werden die Ausgangsstoffe der Formel (I-1-a) und der entsprechende Chlorameisensäureester bzw. Chlorameisensäurethiolester der Formel (VII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (F) ist dadurch gekennzeichnet, daß man Verbindungen der Formel (I-1-a) jeweils mit Verbindungen der Formel (VIII) in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a) ca. 1 Mol Chlormonothioameisensäureester bzw. Chlordithioameisensäureester der Formel (VIII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Nitrile, Ketone, Carbonsäureester, Amide, Sulfone, Sulfoxide, aber auch Halogenalkane.

Vorzugsweise werden Dimethylsulfoxid, Tetrahydrofuran, Ethylacetat, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, daß man Verbindungen der Formel (I-1-a) jeweils mit Sulfonsäurechloriden der Formel (IX) gegebenenfalls in Gegenwart eines Verdünnungsmittels und gegebenenfalls in Gegenwart eines Säurebindemittels umsetzt.

Beim Herstellungsverfahren (G) setzt man pro Mol Ausgangsverbindung der Formel (I-1-a) ca. 1 Mol Sulfonsäurechlorid der Formel (IX) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Ethylacetat, Acetonitril, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (H) ist dadurch gekennzeichnet, daß man Verbindungen der Formel (I-1-a) jeweils mit Phosphorverbindungen der Formel (X) gegebenenfalls in Gegenwart eines Verdünnungsmittels und gegebenenfalls in Gegenwart eines Säurebindemittels umsetzt.

Beim Herstellungsverfahren (H) setzt man zum Erhalt von Verbindungen der Formel (I-1-e) auf 1 Mol der Verbindungen (I-1-a), 1 bis 2, vorzugsweise 1 bis 1,3 Mol der Phosphorverbindung der Formel (X) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Ketone, Carbonsäureester, Nitrile, Alkohole, Sulfide, Sulfone, SulfoXide etc.

Vorzugsweise werden Acetonitril, Dimethylsulfoxid, Ethylacetat, Tetrahydrofuran, Dimethylformamid oder Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (I) ist dadurch gekennzeichnet, daß man Verbindungen der Formel (I-1-a) mit Metallhydroxiden bzw. Metallalkoxiden der Formel (XI) oder Aminen der Formel (XII), gegebenenfalls in Gegenwart eines Verdünnungsmittels, umsetzt.

Als Verdünnungsmittel können bei dem erfindungsgemäßen Verfahren (I) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (I) wird im allgemeinen unter Normaldruck durchgeführt. Die Reaktionstemperaturen liegen im allgemeinen zwischen -20°C und 100°C, vorzugsweise zwischen 0°C und 50°C.

Das erfindungsgemäße Verfahren (J) ist dadurch gekennzeichnet, daß man Verbindungen der Formel (I-1-a) jeweils mit (Jα) Verbindungen der Formel (XIII) gegebenenfalls in Gegenwart eines Verdünnungsmittels und gegebenenfalls in Gegenwart eines Katalysators oder (Jβ) mit Verbindungen der Formel (XIV) gegebenenfalls in Gegenwart eines Verdünnungsmittels und gegebenenfalls in Gegenwart eines Säurebindemittels umsetzt.

Bei Herstellungsverfahren (Jα) setzt man pro Mol Ausgangsverbindung der Formel (I-1-a) ca. 1 Mol Isocyanat der Formel (XIII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Ketone, Carbonsäureester, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (Jβ) setzt man pro Mol Ausgangsverbindung der Formel (I-1-a) ca. 1 Mol Carbamidsäurechlorid der Formel (XIV) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Ketone, Carbonsäureester, Sulfone, Sulfoxide oder halogenierte Kohlenwasserstoffe.

Vorzugsweise werden Dimethylsulfoxid, Tetrahydrofuran, Ethylacetat, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerrettichblattkäfers (Phaedon cochleariae), gegen die Larven der grünen Reiszikade (Nephotettix cincticeps), gegen die Pfirsichblattlaus (Myzus persicae) und auch gegen die Obstbaumspinnmilbe (Panonychus ulmi) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie. Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung, Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diacloden, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren,
Halofenozide, HCH, Heptenophos, Hexaflumuron, Hexythiazox, Hydroprene,
Imidacloprid, Isazofos, Isofenphos, Isoxathion, Ivermectin,
Kernpolyederviren,
Lambda-cyhalothrin, Lufenuron,
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron,
Omethoat, Oxamyl, Oxydemethon M,
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyndaben, Pyridathion, Pyrimidifen, Pyriproxyfen,
Quinalphos,
Ribavirin,
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Theta-cypermethrin, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii,
YI 5302,
Zeta-cypermethrin, Zolaprofos,
(1R-cis)-[5-(Phenylmethyl)-3-furanyl]-methyl-3-[(dihydro-2-oxo-3(2H)-furanyliden)-methyl]-2,2-dimethylcyclopropancarboxylat,
(3-Phenoxyphenyl)-methyl-2,2,3,3-tetramethylcyclopropanecarboxylat,
1-[(2-Chlor-5-thiazolyl)methyl]tetrahydro-3,5-dimethyl-N-nitro-1,3,5-triazin-2(1H)-imin,
2-(2-Chlor-6-fluorphenyl)-4-[4-(1,1-dimethylethyl)phenyl]-4,5-dihydro-oxazol,
2-(Acetlyoxy)-3-dodecyl-1,4-naphthatindion,
2-Chlor-N-[[[4-(1-phenylethoxy)-phenyl]-amino]-carbonyl]-benzwid,
2-Chlor-N-[[[4-(2,2-dichlor-1,1-difluorethoxy)-phenyl]-amino]-carbonyl]-benzamid, 3-Methylphenyl-propylcarbamat.
4-[4-(4-Ethoxyphenyl)-4-methylpentyl]-1-fluor-2-phenoxy-benzol,
4-Chlor-2-(1,1-dimethylethyl)-5-[[2-(2,6-dimethyl-4-phenoxyphenoxy)ethyl]thio]-3(2H)-pyridazinon,
4-Chlor-2-(2-chlor-2-methylpropyl)-5-[(6-iod-3-pyridinyl)methoxy]-3(2H)-pyridazinon,
4-Chlor-5-[(6-chlor-3-pyridinyl)methoxy]-2-(3,4-dichlorphenyl)-3(2H)-pyridazinon,
Bacillus thuringiensis strain EG-2348,
Benzoesäure-[2-benzoyl-1-(1,1-dimethylethyl)-hydrazid,
Butansäure-2,2-dimethyl-3-(2,4-dichlorphenyl)-2-oxo-1-oxaspiro[4.5]dec-3-en-4-yl-ester,
[3-[(6-Chlor-3-pyridinyl)methyl]-2-thiazolidinyliden]-cyanamid,
Dihydro-2-(nitromethylen)-2H-1,3-thiazine-3(4H)-carboxaldehyd,
Ethyl-[2-[[1,6-dihydro-6-oxo-1-(phenylmethyl)-4-pyridazinyl]oxy]ethyl]-carbamat,
N-(3,4,4-Trifluor-1-oxo-3-butenyl)-glycin,
N-(4-Chlorphenyl)-3-[4-(difluormethoxy)phenyl]-4,5-dihydro-4-phenyl-1H-pyrazol-1-carboxamid,
N-[(2-Chlor-5-thiazolyl)methyl]-N'-methyl-N'-nitro-guanidin,
N-Methyl-N'-(1-methyl-2-propenyl)-1,2-hydrazindicarbothioamid
N-Methyl-N'-2-propenyl-1,2-hydrazindicarbothioamid,
O,O-Diethyl-[2-(dipropylamino)-2-oxoethyl]-ethylphosphoramidothioat.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Arytoxy-phenoxy-alkansäureester, wie z.B. Diclofop-methyt Fenoxaprop-ethyl, Fluazifop-butyl, Haloxyfopmethyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuronmethyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel 1 eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze
   wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösung- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw.. modifiziertes Alkydharz, Phenolharz, Kohlenwasserstoffbarz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixieruagsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron sowie Fungizide wie Epoxyconazole, Hexaconazole, Azaconazole, Propiconazole, Tebuconazole, Cyproconazole, Metconazole, Imazalil, Dichlofluanid, Tolylfluanid, 3-Iod-2-propinyl-butylcarbamat, N-Octyl-isothiazolin-3-on und 4,5-Dichlor-N-octylisothiazolin-3-on genannt.

Die. Herstellung und die Verwendung der erfindungsgemäßen Wirkstoffe gehen aus den nachfolgenden Beispielen hervor.

Die Großbuchstaben in einigen der Beispielnummern sollen zum leichteren Auffinden dieser Strukturen in den Formelschemata dienen. Sie bezeichnen unterschiedliche Bedeutungen von Q.

### Beispiele

### Beispiel I-1-a-1

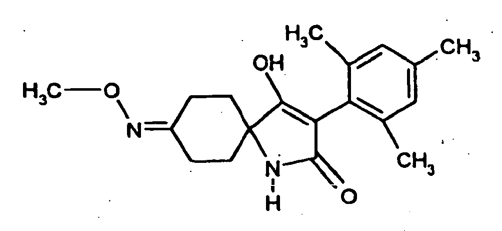

Zu 7,8 g Kalium-tert.-butylat in 20 ml wasserfreiem DMF (Dimethylformamid) gibt man bei 60°C 7,2 g der Verbindung gemäß Beispiel IIC-1 in 10 ml wasserfreiem DMF. Man rührt bei dieser Temperatur, bis die Reaktion beendet ist (dünnschichtchromatographische Kontrolle (DC), Laufmittel Methylenchlorid:Essigester 5:3), engt im Vakuum ein, nimmt den Rückstand in 100 ml Wasser auf und säuert bei ca. 0°C mit 20 %iger Salzsäure an. Der Nederschlag wird abgesaugt und getrocknet.
Ausbeute 4,0 g (61 % der Theorie), Fp. 250°C.

### Beispiel I-1-a-2

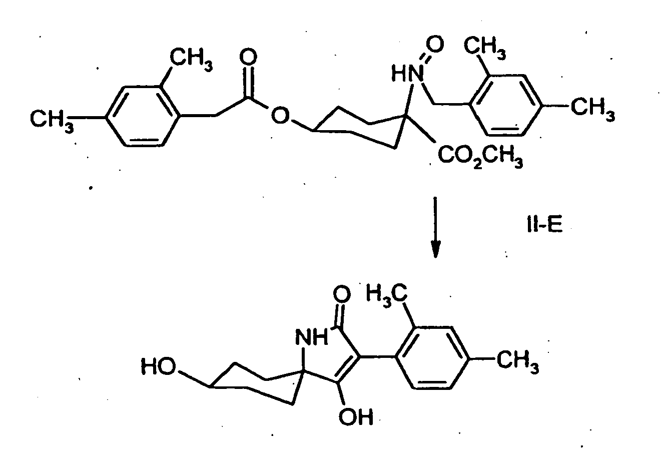

In 50 ml DMF werden 12 g Kalium-tert.-butylat und 5,3 g der Verbindung II-E 2 Stunden bei 80°C gerührt. Man versetzt mit Toluol und engt im Vakuum ein. Der Rückstand wird in 30 ml Eiswasser aufgenommen und bei ca. 0°C mit 20 %iger Salzsäure versetzt, bis der pH-Wert etwa zwischen 5 und 6 liegt. Dann wird abgesaugt und säulenchromatografisch gereinigt (Kieselgel, Methylenchlorid/Essigsäureethylester 5/3). Ausbeute 1,15 g (36 % der Theorie), Fp. 163°C.

### Beispiel I-1-a-3

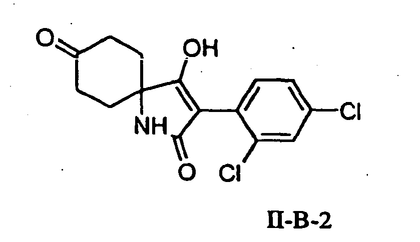

Analog erhält man ausgehend von der Verbindung gemäß Beispiel II-B-2 die oben gezeigte Verbindung vom Fp. > 250°C.

### Beispiel I-1-a-4

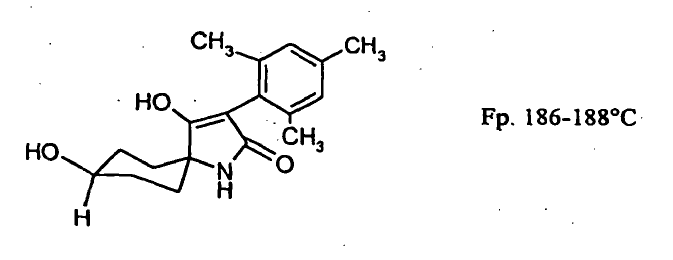

Nach den zuvor angegebenen Methoden werden auch die in der folgenden Tabelle 25 aufgeführten Stoffe der Formel (I-1-a) hergestellt.

**Tabelle 25**

| | | | | | | | |
|---|---|---|---|---|---|---|---|
| 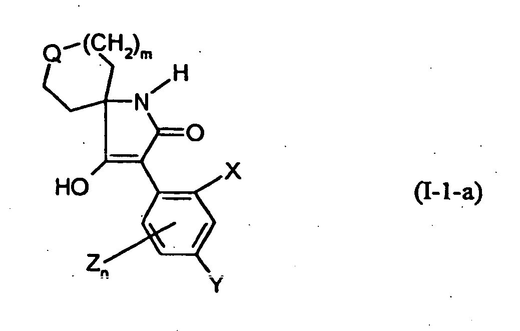 | | | | | | | |

| **Bsp.-Nr.** | **X** | **Y** | | **Zₙ** | **Q** | | **Schmelzpunkt in °C** |
|---|---|---|---|---|---|---|---|
| I-1-a-5 | CH₃ | H | | 5-CH₃ | 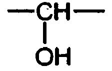 | 1 | 143 |
| I-1-a-6 | CH₃ | CH₃ | | 3,6-(CH₃)₂ | 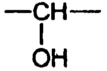 | 1 | >250 |
| I-1-a-7 | CH₃ | H | | 5-CH₃ | 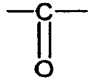 | 1 | |
| I-1-a-8 | CH₃ | CH₃ | | 3,6-(CH₃)₂ | 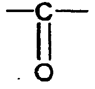 | 1 | |

### Beispiel I-1-b-1

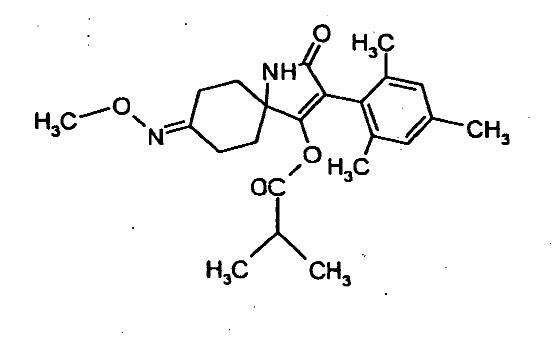

2 g der Verbindung gemäß Beispiel I-1-a-1 werden mit 1,1 ml Triethylamin und 0,75 g iso-Buttersäurechlorid 4 Stunden in 50 ml Essigsäureethylester (Essigester) unter Rückfluß gekocht. Man engt ein und chromatographiert den Rückstand an Kieselgel (Laufmittel Hexan:Aceton 7:3).
Ausbeute 1,15 g (48 % der Theorie), Fp. 247°C.

### Beispiel I-1-b-2

Analog zu Beispiel I-1-b-1 erhält man die folgende Verbindung vom Fp. 189°C.

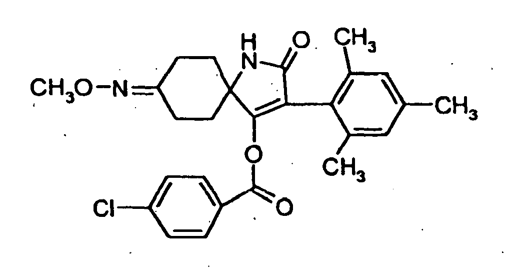

### Beispiel I-1-c-1

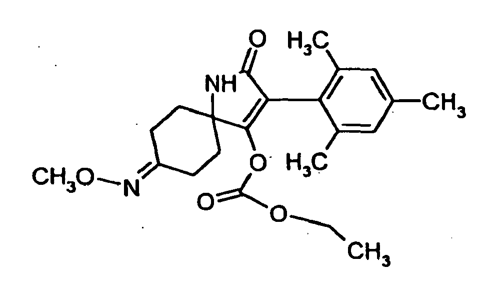

In 1,3 g der Verbindung gemäß Beispiel (I-1-a-1) und 0,6 ml Triethylamin in 50 ml wasserfreiem Methylenchlorid tropft man bei 0°C 0,4 ml Chlorameisensäureethylester in 5 ml wasserfreiem Methylenchlorid und rührt noch einen Tag ohne Kühlung. Dann wäscht man zweimal mit 50ml 0,5 N NaOH, trocknet und engt ein. Ausbeute 0,70 g (45 % der Theorie), Fp. 196°C.

### Beispiel II-B-1

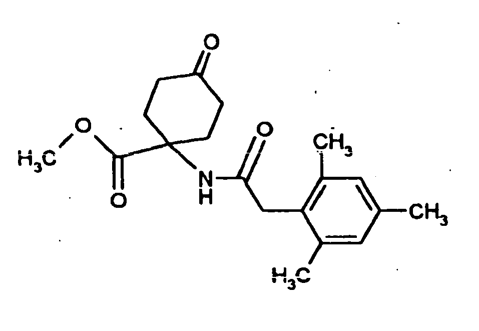

Zu 75 g konzentrierter Schwefelsäure tropft man bei einer Innentemperatur von 30 bis 40°C eine Suspension von 50 g der Verbindung gemäß Beispiel XX-C-1 und rührt noch 2 Stunden bei dieser Temperatur. Dann tropft man 110 ml Methanol so zu, daß sich eine Innentemperatur von 40°C einstellt. Anschließend rührt man noch 6 Stunden bei 40 bis 70°C. Man gießt auf 600 g Eis, versetzt mit wäßriger NaHCO₃-Lösung und extrahiert mit Methylenchlorid.
Ausbeute 40 g (74 % der Theorie), Fp. 136-137°C.

### Beispiel II-B-2

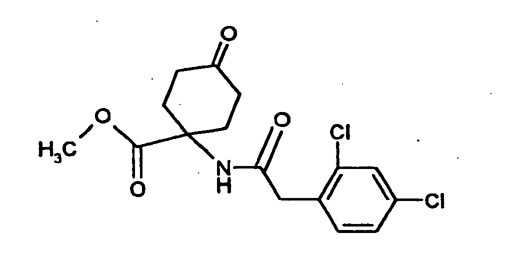

Analog zu Beispiel II-B-1 erhält man aus 69 g der Verbindung gemäß Beispiel XX-B-1 11,7 g (15 % der Theorie) der oben gezeigten Verbindung.
Fp. 118-120°C.

### Beispiel II-C-1

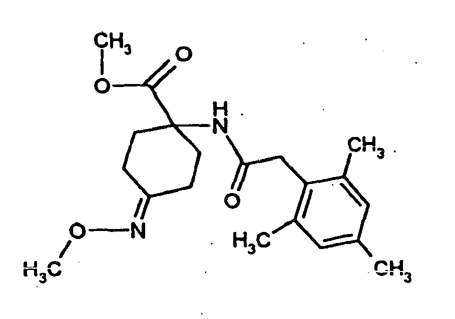

20 g der Verbindung gemäß Beispiel II-B-1 in 50 ml Pyridin werden bei Raumtemperatur mit 4,2 g O-Methylhydroxylaminhydrochlorid versetzt und 4 Stunden bei 50°C gerührt. Man engt im Vakuum ein, versetzt den Rückstand mit ca. 50 ml Wasser und extrahiert 3 mal mit Methylenchlorid. Die vereinigten organischen Phasen werden getrocknet und eingedampft. Der Rückstand wird mit Methylenchlorid:Essigester 5:3 an Kieselgel chromatographiert.
Ausbeute 19,9 g (99 % der Theorie), Fp. 158-160°C.

### Beispiel II-E-1

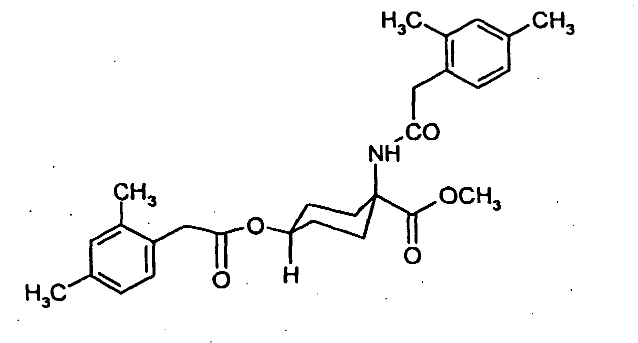

32 g der Verbindung der Formel (XV) 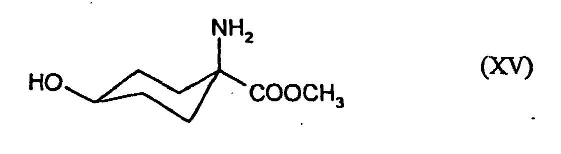 und 57 ml Triethylamin in 200 ml Tetrahydrofuran werden bei -10 bis 0°C mit 34 g 2,4-Dimethylphenylessigsäurechlorid versetzt und noch einen Tag bei Raumtemperatur gerührt. Man saugt ab und engt das Filtrat ein. Der Rückstand wird an Kieselgel chromatografiert. Ausbeute 5,3 g.
¹H-NMR (400 MHz, d₆-DMSO): δ = 2,19-2,25 (4s, 12H, Ar CH₃), 4,63-4,69, 4,82-4,86 (m, 1H, CO-O-CH), 6,9-7,06 (m, 6H, Ar-H)

### Beispiel XIX-1

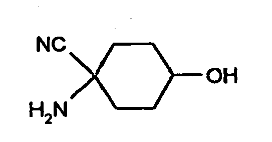

Zum Gemisch von 414,2 g 25 %iger Ammoniaklösung, 139,6 g Ammoniumchlorid, 127,9 g Natriumcyanid und 392 ml Wasser tropft man bei Raumtemperatur 248,3 g 4-Hydroxycyclohexanon und rührt über Nacht bei 45°C. Der Niederschlag wird abgesaugt und getrocknet.
Ausbeute 197 g (64 % der Theorie), Fp. 130°C.

### Beispiel XIX-2

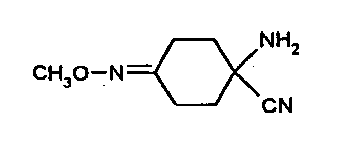

Zum Gemisch aus 48,5 g 25 %ige Ammoniaklösung, 16,4 g Ammoniumchlorid, 15,0 g Natriumcyanid in 46 ml Wasser tropft man bei Raumtemperatur 36,0 g der Verbindung gemäß Beispiel XXV-1 und rührt über Nacht bei 38°C. Nach Extraktion und Methylenchlorid und üblicher Aufarbeitung erhält man 33,8 g (79 % der Theorie) der oben gezeigten Verbindung als Öl.

### Beispiel XX-A-1

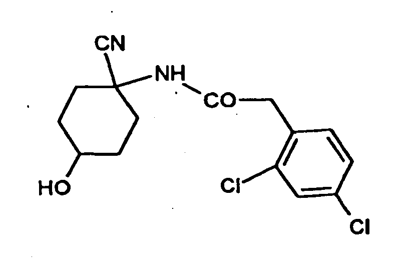

Zu 49 g 1-Cyano-4-hydroxycyclohexylamin gemäß Beispiel XIX-1 und 49 ml Triethylamin in 500 ml THF gibt man bei -20°C 79 g 2,4-Dichlorphenylessigsäurechlorid in 50 ml THF. Man rührt 1 Tag bei Raumtemperatur, extrahiert mit 1 1 0,5N HCl, trocknet die organische Phase und engt sie ein.
Ausbeute 70 g (61 % der Theorie), Fp. 148-150°C.

### Beispiel XX-B-1

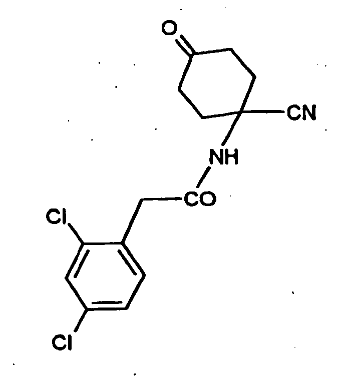

70 g der Verbindung gemäß Beispiel XX-A-1 werden analog zu Beispiel XXV-1 oxidiert.
Ausbeute 69,0 g (99 % der Theorie), Fp. 76-78°C.

### Beispiel XX-C-1

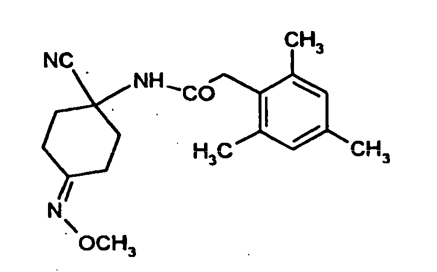

Zu 33 g der Verbindung gemäß Beispiel XIX-1 und 32 ml Triethylamin in 100 ml THF (Tetrahydrofuran) gibt man bei etwa 0°C 40 g 2,4,6-Trimethylphenylessigsäurechlorid in 50 ml THF und rührt 2 Stunden bei Raumtemperatur. Zur Aufarbeitung wird das Reaktionsgemisch mit 1 l 1N NaOH geschüttelt, die organische Phase abgetrennt, getrocknet und eingedampft.
Ausbeute 52 g (79 % der Theorie), Fp. 224°C.

### Beispiel XXV-1

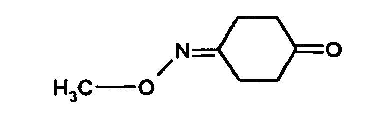

In 300 ml Methylenchlorid legt man bei -70°C 11 ml Oxalylchlorid vor. Man gibt 10 ml DMSO (Dimethylsulfoxid) zu und rührt 3 Minuten bei -35°C. Man kühlt wieder auf -70°C und gibt innerhalb 1 Stunde 150 g der Verbindung gemäß Beispiel XXVI-1 als 10 %ige Lösung in Methylenchlorid zu. Man rührt noch 15 Minuten bei -35°C, gibt dann 130 ml Triethylamin zu und rührt 1 Stunde bei Raumtemperatur. Dann versetzt man vorsichtig mit Wasser, extrahiert mit Methylenchlorid und filtriert über Kieselgel.
Ausbeute 7,9 g (56 % der Theorie), Öl.

### Beispiel XXVI-1

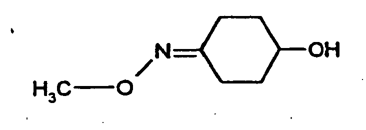

Bei Raumtemperatur versetzt man 5,7 g 4-Hydroxycyclohexanon in 50 ml Pyridin mit 4,2 g O-Methylhydroxylaminhydrochlorid und rührt 4 Stunden bei 50°C. Man engt im Vakuum ein, nimmt den Rückstand in ca. 50 ml Wasser auf und extrahiert 3 mal mit Methylenchlorid. Die vereinigten organischen Phasen werden getrocknet und eingeengt.
Ausbeute 6,9 g (96 % der Theorie), Öl.

### Beispiel XXVII-1

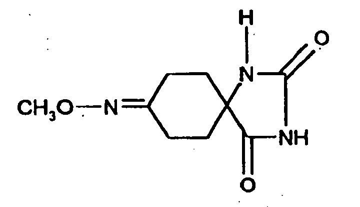

Zu 446,4 g Ammoniumcarbonat und 98,0 g Natriumcyanid in 1600 ml Wasser gibt man eine Lösung von 70,5 g der Verbindung gemäß Beispiel XXV-1 in 1600 ml Ethanol und rührt 10 Stunden bei 60°C. Dann kühlt man auf 5°C und saugt den Niederschlag ab.
Ausbeute 156,7 g (74 % der Theorie), Fp. >250°C.

### Anwendungsbeispiele

### Beispiel A

### Myzus-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus *(Myzus persicae)* befallen sind, werden durch Tauchen in die Wirkstoffzubereitung der gewünschten Konzentration behandelt.

Nach der gewünschten Zeit wird die Abtötung in Prozent bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß der Herstellungsbeispiele I-1-a-1 und I-1-b-1 bei einer beispielhaften Wirkstoffkonzentration von 0,1 % eine Abtötung von jeweils 100 % nach 6 Tagen.

### Beispiel B

### Nephotettix-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade *(Nephotettix cincticeps)* besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen I-1-a-1 und I-1-b-1 bei einer beispielhaften Wirkstoffkonzentration von 0,1 % eine Abtötung von jeweils 100 % nach 6 Tagen.

### Beispiel C

### Panonychus-Test

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Ca. 30 cm hohe Pflaumenbäumchen (Prunus domestica), die stark von allen Entwicklungsstadien der Obstbaumspinnmilbe *(Panonychus ulmi)* befallen sind, werden mit einer Wirkstoffzubereitung der gewünschten Konzentration gespritzt.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test hatte z.B. die Verbindung gemäß Herstellungsbeispiel bei einer beispielhaften Wirkstoffkonzentration von 0,02 % eine Wirkung von 100 % nach 7 Tagen.

### Beispiel D

### Phaedon-Larven-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers *(Phaedon cochleariae)* besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen I-1-a-1 und I-1-b-1 bei einer beispielhaften Wirkstoffkonzentration von 0,1 % eine Abtötung von jeweils 100 % nach 7 Tagen.

### Beispiel E

### Spodoptera Frugiperda-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirksoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters *(Spodoptera frugiperda)* besetzt, solange die Blätter noch feucht sind.

Nach der gewünschten Zeit wird die in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen I-1-a-1 und I-1-b-1 bei einer, beispielhaften Wirkstoffkonzentration von 0,1 % eine Abtötung von jeweils 100 % nach 7 Tagen.

### Beispiel F

### Tetranychus-Test (OP-resistent/Tauchbehandlung)

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Stadien der gemeinen Spinnmilbe *(Tetranychus urticae)* befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test bewirkten z.B. die Verbindungen gemäß den Herstellungsbeispielen I-1-b-1 bei einer beispielhaften Wirkstoffkonzentration von 0,01 % jeweils eine Wirkung von 100% nach 7 Tagen.

### Beispiel G

### Test mit Boophilus microplus resistent/SP-resistenter Parkhurst-Stamm

| | |
|---|---|
| Testtiere: | adulte gezogene Weibchen |
| Lösungsmittel: | Dimethylsulfoxid |

20 mg Wirkstoff werden in 1 ml Dimethylsulfoxid gelöst, geringere Konzentrationen werden durch Verdünnen in dem gleichen Lösungsmittel hergestellt.

Der Test wird in 5-fach-Bestimmung durchgeführt. 1 µl der Lösungen wird in das Abdomen injiziert, die Tiere in Schalen überführt und in einem klimatisierten Raum aufbewahrt. Die Wirkungskontrolle erfolgt nach 7 Tagen auf Hemmung der Eiablage. Eine Wirkung von 100 % bedeutet, daß keine Zecke Eier gelegt hat.

In diesem Test hatten z.B. die Verbindungen gemäß Herstellungsbeispielen I-1-a-1 und I-1-b-1 bei einer beispielhaften Wirkstoffkonzentration von 20 µg/Tier jeweils eine Wirkung von 100 %.

### Beispiel H

### Blowfly-Larven-Test/Entwicklungshemmende Wirkung

| | |
|---|---|
| Testtiere: | Lucilia cuprina-Larven |
| Lösungsmittel: | Dimethylsulfoxid |

20 mg Wirkstoff werden in 1 ml Dimethylsulfoxid gelöst, geringere Konzentrationen werden durch Verdünnen mit dest. H₂O hergestellt.

Etwa 20 Lucilia cuprina-Larven werden in ein Teströhrchen gebracht, welches ca. 1 cm³ Pferdefleisch und 0,5 ml der zu testenden Wirkstoffzubereitung enthält. Nach 24 und 48 Stunden wird die Wirksamkeit der Wirkstoffzubereitung ermittelt. Die Teströhrchen werden in Becher mit Sand-bedecktem Boden überführt. Nach weiteren 2 Tagen werden die Teströhrchen entfernt und die Puppen ausgezählt.

Die Wirkung der Wirkstoffzubereitung wird nach der Zahl der geschlüpften Fliegen nach 1,5-facher Entwicklungsdauer einer unbehandelten Kontrolle beurteilt. Dabei bedeutet 100 %, daß keine Fliegen geschlüpft sind; 0 % bedeutet, daß alle Fliegen normal geschlüpft sind.

In diesem Test hatten z.B. die Verbindungen gemäß den Herstellungsbeispielen I-1-a-1 bei einer beispielhaften Wirkstoffkonzentration von 1000 ppm jeweils eine Wirkung von 100 %.

## Patentansprüche

1. Verbindungen der Formel (I) 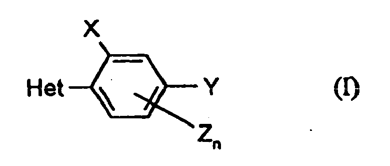 in welcher
X für Chlor, Methyl, steht,
Y für Wasserstoff, Chlor, Methyl, steht,
Z für Chlor, Methyl, steht,
n für 0, 1 oder 2 steht,
Het für eine der Gruppen 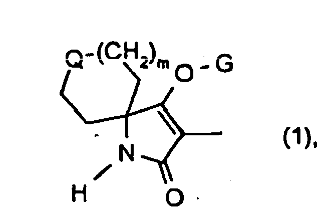 steht,
G für Wasserstoff (a) oder für eine der Gruppen 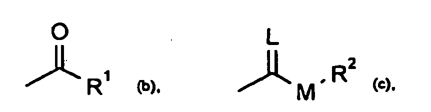 steht,
worin
L für Sauerstoff steht und
M für Sauerstoff steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl oder C₁-C₄-Alkylthio-C₁-C₆-alkyl oder für gegebenenfalls durch Methyl, Ethyl, tert.-Butyl oder Methoxy substituiertes C₃-C₆-Cycloalkyl,
für gegebenenfalls durch Fluor, Chlor, Brom, Cyano, Nitro, Methyl, Methoxy, Trifluormethyl oder Trifluormethoxy substituiertes Phenyl,
für gegebenenfalls durch Fluor, Chlor, Brom, Methyl, Methoxy, Trifluormethyl oder Trifluormethoxy substituiertes Benzyl oder
für jeweils gegebenenfalls durch Fluor, Chlor, Brom oder Methyl substituiertes Furanyl, Thienyl oder Pyridyl steht,
R² für C₁-C₁₄-Alkyl, C₂-C₁₄-Alkenyl oder C₁-C₄-Alkoxy-C₂-C₆-alkyl,
für gegebenenfalls durch Methyl oder Methoxy substituiertes C₃-C₆-Cycloalkyl oder
für jeweils gegebenenfalls durch Fluor, Chlor, Brom, Cyano, Nitro, Methyl, Methoxy, Trifluormethyl oder Trifluormethoxy substituiertes Phenyl oder Benzyl steht,
Q für 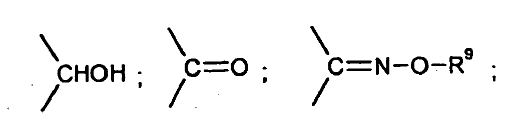 oder 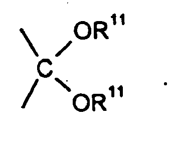 steht,
R⁹ für Wasserstoff, für C₁-C₄-Alkyl, für C₃-C₆-Cycloalkyl oder für jeweils gegebenenfalls durch Fluor, Chlor, Brom, Methyl, Ethyl, n-Propyl, iso-Propyl, tert.-Butyl, Methoxy, Trifluormethyl, Trifluormethoxy, Nitro oder Cyano substituiertes Phenyl oder Benzyl oder für CO-R^{1'}, CO₂R^{2'}, SO₂R^{1'}, CONH₂, CONHR¹¹ oder 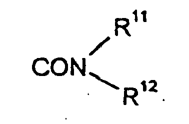 steht,
R¹¹ und R¹² gleich oder verschieden sind und für Methyl oder Ethyl steht,
m für 1 steht,
R^{1'} unabhängig von R¹ für die oben für R¹ als ganz besonders bevorzugt genannten Bedeutungen steht und
R^{2'} unabhängig von R² für die oben für R² als ganz besonders bevorzugt genannten Bedeutungen steht.

2. Verfahren zur Herstellung von Verbindungen der Formel (I) gemäß Anspruch 1, **dadurch gekennzeichnet, daß** man zum Erhalt von Verbindungen
(A) der Formel (I-1-a) 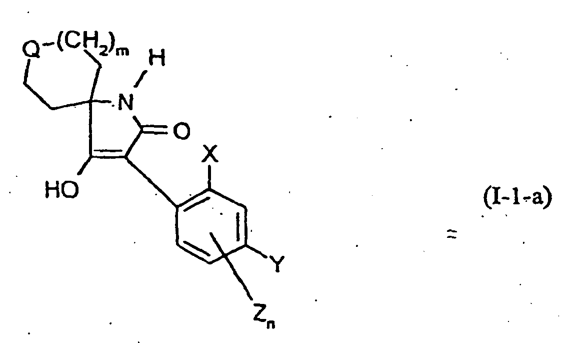 in welcher
Q, X, Y, Z, m und n die in Anspruch 1 angegebenen Bedeutungen haben,
Verbindungen der Formel (II) 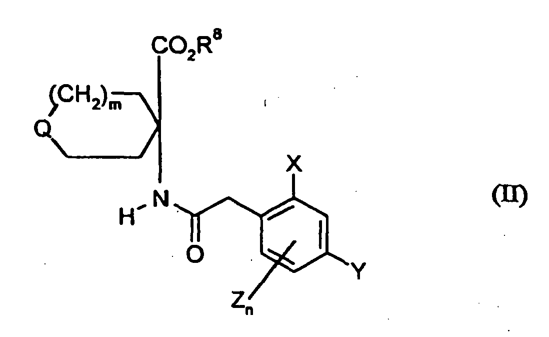 in welcher
Q, X, Y, Z, m und n die oben angegebenen Bedeutungen haben,
und
R⁸ für Alkyl steht,
in Gegenwart eines Verdünnungsmittels und in Gegenwart einer Base intramolekular kondensiert,
und gegebenenfalls anschließend die so erhaltenen Verbindungen der Formeln (I-1-a), jeweils
(Dα) mit Säurechloriden der Formel (V) 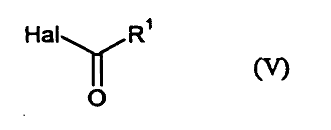 in welcher
R¹ die in Anspruch 1 angegebenen Bedeutungen hat und
Hal für Halogen steht
oder
(β) mit Carbonsäureanhydriden der Formel (VI)
R¹-CO-O-CO-R¹ (VI)
in welcher
R¹ die oben angegebenen Bedeutungen hat,
gegebenenfalls in Gegenwart eines Verdünnungsmittels und gegebenenfalls in Gegenwart eines Säurebindemittels umsetzt; oder
(E) mit Chlorameisensäureestern oder Chlorameisensäurethiolestern der Formel (VII)
R²-M-CO-Cl (VII)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt; oder
(F) mit Chlormonothioameisensäureestern oder Chlordithioameisensäureestern der Formel (VIII) 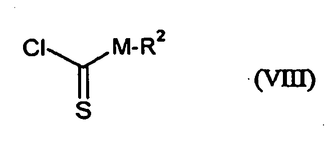 in welcher
M und R² die oben angegebenen Bedeutungen haben,
gegebenenfalls in Gegenwart eines Verdünnungsmittels und gegebenenfalls in Gegenwart eines Säurebindemittels umsetzt; oder
(G) mit Sulfonsäurechloriden der Formel (IX)
R³-SO₂-Cl (IX)
in welcher
R³ die in Anspruch 1 angegebene Bedeutung hat,
gegebenenfalls in Gegenwart eines Verdünnungsmittels und gegebenenfalls in Gegenwart eines Säurebindemittels umsetzt; oder
(H) mit Phosphorverbindungen der Formel (X) 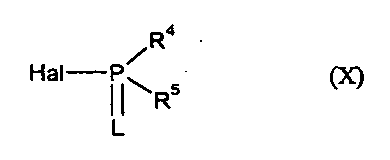 in welcher
L, R⁴ und R⁵ die in Anspruch 1 angegebenen Bedeutungen haben und
Hal für Halogen steht,
gegebenenfalls in Gegenwart eines Verdünnungsmittels und gegebenenfalls in Gegenwart eines Säurebindemittels umsetzt; oder
(I) mit Metallverbindungen oder Aminen der Formeln (XI) oder (XII)
Me(OR¹³)ₜ (XI) 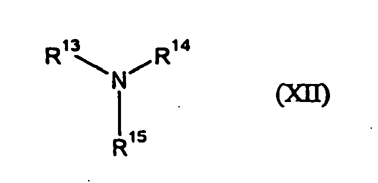
in welchen
Me für ein ein- oder zweiwertiges Metall,
t für 1 oder 2 und
R¹³, R¹⁴, R¹⁵ unabhängig voneinander für Wasserstoff oder Alkyl stehen,
gegebenenfalls in Gegenwart eines Verdünnungsmittels umsetzt; oder
(Jα) mit Isocyanaten oder Isothiocyanaten der Formel (XIII)
R⁶-N=C=L (XIII)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XIV) 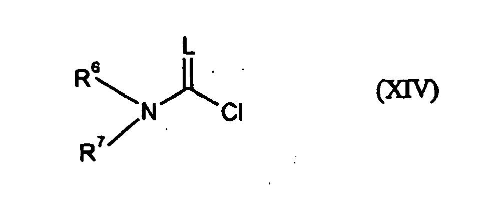 in welcher
L, R⁶ und R⁷ die in Anspruch 1 angegebenen Bedeutungen haben,
gegebenenfalls in Gegenwart eines Verdünnungsmittels und gegebenenfalls in Gegenwart eines Säurebindemittels, umsetzt.

3. Verbindungen der Formel (II) 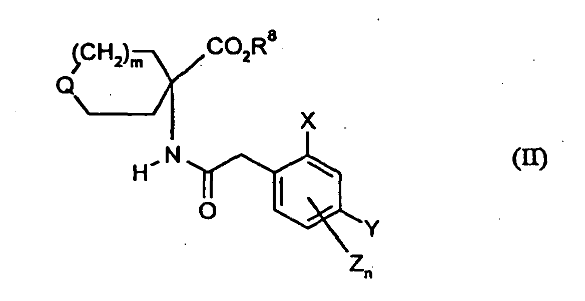 in welcher
Q, X, Y, Z, m und n die oben angegebenen Bedeutungen haben und
R⁸ für Alkyl steht.

4. Verbindungen der Formel (XVII) 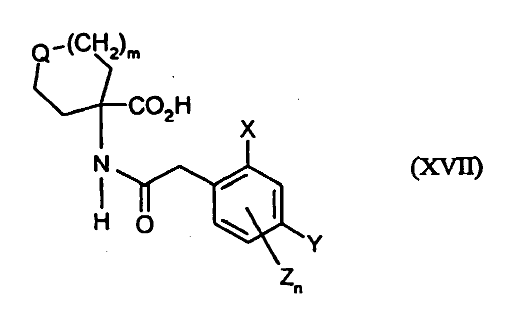 in welcher
Q, m, X, Y, Z und n die in Anspruch 1 angegebenen Bedeutungen haben.

5. Verbindungen der Formel (XX) 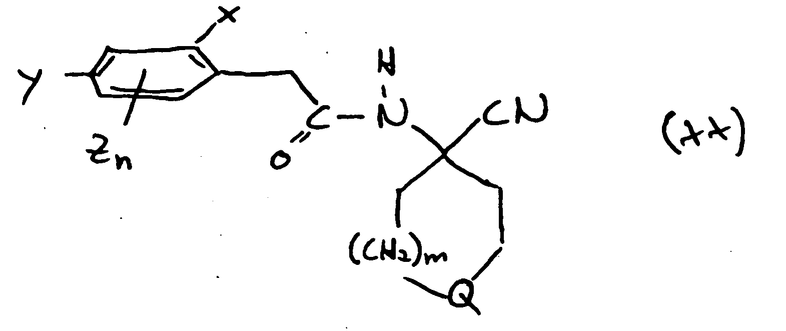 in welcher
X, Y, Z, n, m und Q die in Anspruch 1 angegebene Bedeutung haben.

6. Schädlingsbekämpfungsmittel oder herbizide Mittel, **gekennzeichnet durch** einen Gehalt an einer Verbindung der Formel (I) gemäß Anspruch 1.

7. Verwendung von Verbindungen der Formel (I) gemäß Anspruch 1 zur Bekämpfung von Schädlingen und Unkräutern.

8. Verfahren zur Bekämpfung von Schädlingen und Unkräutern, **dadurch gekennzeichnet, daß** man Verbindungen der Formel (I) gemäß Anspruch 1 auf Schädlinge bzw. Unkräuter und/oder ihren Lebensraum einwirken läßt.

9. Verfahren zur Herstellung von Schädlingsbekämpfungsmitteln und herbiziden Mitteln, **dadurch gekennzeichnet, daß** man Verbindungen der Formel (I) gemäß Anspruch 1 mit Streckmitteln und/oder oberflächenaktiven Mitteln vermischt.

## Claims

1. Compounds of the formula (I) 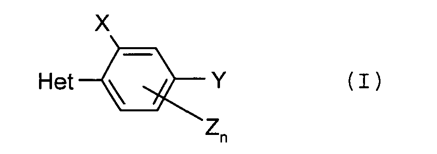 in which
X represents chlorine, methyl,
Y represents hydrogen, chlorine, methyl,
Z represents chlorine, methyl,
n represents 0, 1 or 2,
Het represents one of the groups 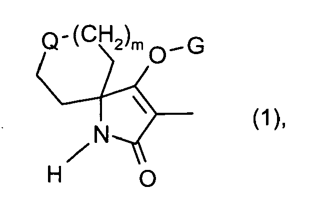
G represents hydrogen (a) or represents one of the groups 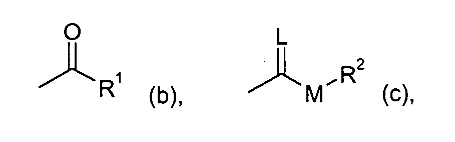 in which
L represents oxygen and
M represents oxygen,
R¹ represents C₁-C₁₄-alkyl, C₂-C₁₄-alkenyl, C₁-C₄-alkoxy-C₁-C₆-alkyl or C₁-C₄-alkylthio-C₁-C₆-alkyl, each of which is optionally substituted by fluorine or chlorine, or represents C₃-C₆-cycloalkyl, which is optionally substituted by methyl, ethyl, tert-butyl or methoxy,
represents phenyl which is optionally substituted by fluorine, chlorine, bromine, cyano, nitro, methyl, methoxy, trifluoromethyl or trifluoromethoxy,
represents benzyl which is optionally substituted by fluorine, chlorine, bromine, methyl, methoxy, trifluoromethyl or trifluoromethoxy or
represents furanyl, thienyl or pyridyl, each of which is optionally substituted by fluorine, chlorine, bromine or methyl,
R² represents C₁-C₁₄-alkyl, C₂-C₁₄-alkenyl or C₁-C₄-alkoxy-C₂-C₆-alkyl,
represents C₃-C₆-cycloalkyl which is optionally substituted by methyl or methoxy or
represents phenyl or benzyl, each of which is optionally substituted by fluorine, chlorine, bromine, cyano, nitro, methyl, methoxy, trifluoromethyl or trifluoromethoxy,
Q represents 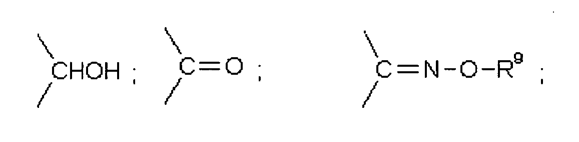 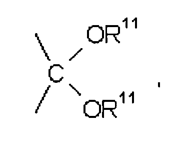
R⁹ represents hydrogen, represents C₁-C₄-alkyl, represents C₃-C₆-cycloalkyl or represents phenyl or benzyl, each of which is optionally substituted by fluorine, chlorine, bromine, methyl, ethyl, n-propyl, iso-propyl, tert-butyl, methoxy, trifluoromethyl, trifluoromethoxy, nitro or cyano, or represents CO-R^{1'}, CO₂R^{2'}, SO₂R^{1'}, CONH₂, CONHR¹¹ or 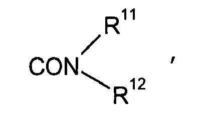
R¹¹ and R¹² are identical or different and each represents methyl or ethyl,
m represents 1,
R^{1'} independently of R¹ has the meanings given above as being very particularly preferred for R¹ and
R²' independently of R² has the meanings given above as being very particularly preferred for R².

2. Process for preparing compounds of the formula (I) according to Claim 1, **characterized in that** to obtain compounds
(A) of the formula (I-1-a) 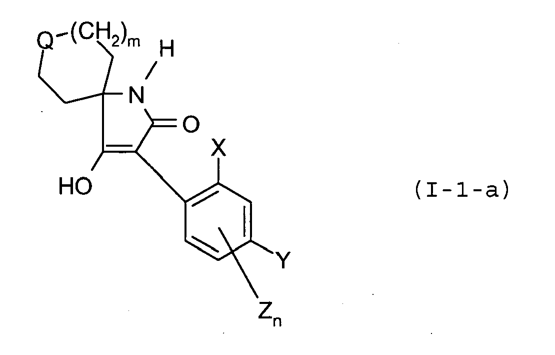 in which
Q, X, Y, Z, m and n are each as defined in Claim 1,
compounds of the formula (II) 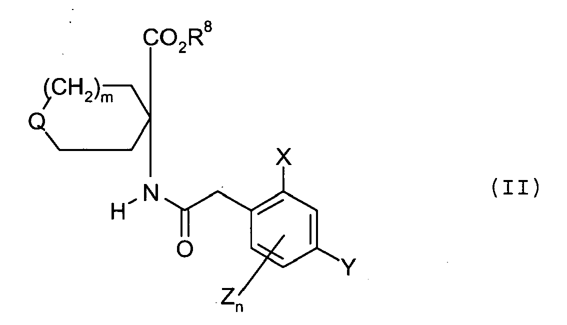 in which
Q, X, Y, Z, m and n above,
and
are each as defined
R⁸ represents alkyl
are intramolecularly condensed in the presence of a diluent and in the presence of a base,
and the resulting compounds of the formula (I-1-a) are each optionally subsequently
(Dα) reacted with acyl chlorides of the formula (V) 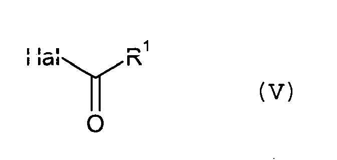 in which
R¹ is as defined in Claim 1 and
Hal represents halogen
or
(β) reacted with carboxylic anhydrides of the formula (VI)
R¹-CO-O-CO-R¹ (VI)
in which
R¹ is as defined above,
if appropriate in the presence of a diluent and if appropriate in the presence of an acid binder; or
(E) reacted with chloroformic acid esters or chloroformic acid thiol esters of the formula (VII)
R²-M-CO-Cl (VII)
in which
R² and M are each as defined in Claim 1,
if appropriate in the presence of a diluent and if appropriate in the presence of an acid binder; or
(F) reacted with chloromonothioformic acid esters or chlorodithioformic acid esters of the formula (VIII) 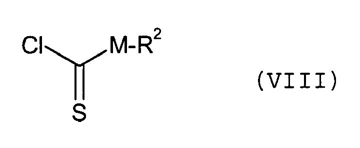 in which
M and R² are each as defined above,
if appropriate in the presence of a diluent and if appropriate in the presence of an acid binder; or
(G) reacted with sulphonyl chlorides of the formula (IX)
R³-SO₂-Cl (IX)
in which
R³ is as defined in Claim 1,
if appropriate in the presence of a diluent and if appropriate in the presence of an acid binder; or
(H) reacted with phosphorus compounds of the formula (X) 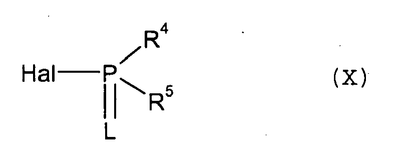 in which
L, R⁴ and R⁵ are each as defined in Claim 1 and
Hal represents halogen,
if appropriate in the presence of a diluent and if appropriate in the presence of an acid binder; or
(I) reacted with metal compounds or amines of the formula (XI) or (XII)
Me(OR¹³)ₜ (XI) 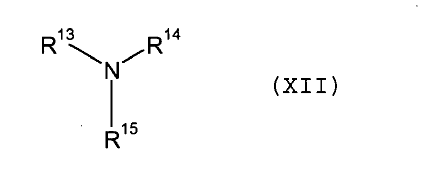
in which
Me represents a mono- or divalent metal,
t represents 1 or 2 and
R¹³, R¹⁴, R¹⁵ independently of one another each represent hydrogen or alkyl,
if appropriate in the presence of a diluent; or
(Jα) reacted with isocyanates or isothiocyanates of the formula (XIII)
R⁶-N=C=L (XIII)
in which
R⁶ and L are each as defined in Claim 1,
if appropriate in the presence of a diluent and if appropriate in the presence of a catalyst or
(β) reacted with carbamoyl chlorides or thiocarbamoyl chlorides of the formula (XIV) 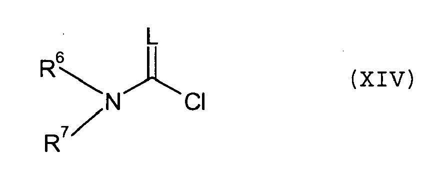 in which
L, R⁶ and R⁷ are each as defined in Claim 1,
if appropriate in the presence of a diluent and if appropriate in the presence of an acid binder.

3. Compounds of the formula (II) 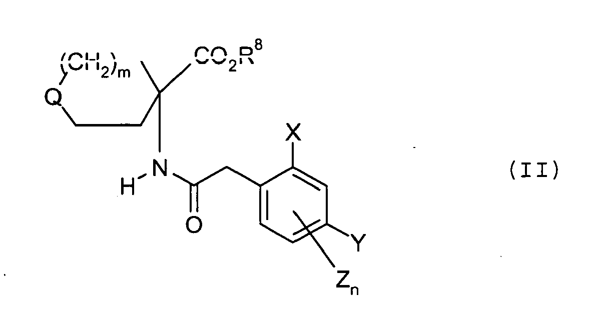 in which
Q, X, Y, Z, m and n are each as defined above and
R⁸ represents alkyl.

4. Compounds of the formula (XVII) 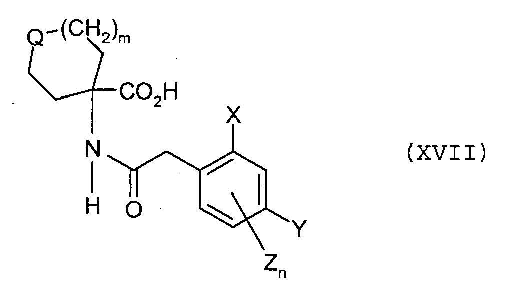 in which
Q, m, X, Y, Z and n are each as defined in Claim 1.

5. Compounds of the formula (XX) 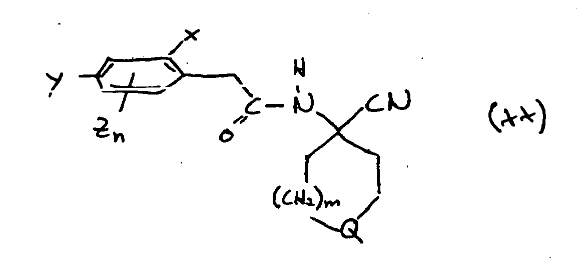 in which
X, Y, Z, n, m and Q are each as defined in Claim 1.

6. Pesticides or herbicides, **characterized in that** they contain a compound of the formula (I) according to Claim 1.

7. The use of compounds of the formula (I) according to Claim 1 for controlling pests and weeds.

8. Method for controlling pests and weeds, **characterized in that** compounds of the formula (I) according to Claim 1 are allowed to act on pests and weeds, respectively, and/or their habitat.

9. Process for preparing pesticides and herbicides, **characterized in that** compounds of the formula (I) according to Claim 1 are mixed with extenders and/or surfactants.

## Revendications

1. Composés de formule (I) 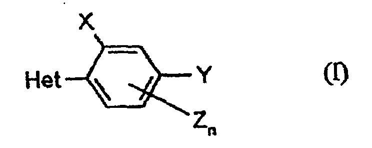 dans laquelle
X représente le chlore, un reste méthyle,
Y représente l'hydrogène, le chlore, un reste méthyle,
Z représente le chlore, un reste méthyle,
n a la valeur 0, 1 ou 2,
Het représente le groupe 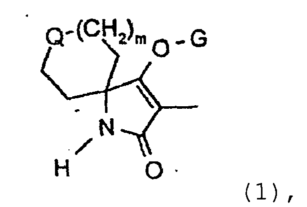
G représente l'hydrogène (a) ou l'un des groupes 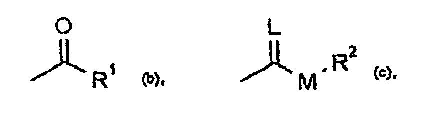 où
L représente l'oxygène et
M représente l'azote,
R¹ est un reste alkyle en C₁ à C₁₄, alcényle en C₂ à C₁₄, (alkoxy en C₁ à C₄)-(alkyle en C₁ à C₆) ou (alkylthio en C₁ à C₄)-(alkyle en C₁ à C₆), chacun éventuellement substitué par du fluor ou par du chlore, ou bien un reste cycloalkyle en C₃ à C₆ éventuellement substitué par un radical méthyle, éthyle, tertiobutyle ou méthoxy,
un reste phényle éventuellement substitué par un radical fluoro, chloro, bromo, cyano, nitro, méthyle, méthoxy, trifluorométhyle ou trifluorométhoxy,
un reste benzyle éventuellement substitué par un radical fluoro, chloro, bromo, méthyle, méthoxy, trifluorométhyle ou trifluorométhoxy, ou bien
un reste furannyle, thiényle ou pyridyle, chacun éventuellement substitué par un radical fluoro, chloro, bromo ou méthyle,
R² est un reste alkyle en C₁ à C₄, alcényle en C₂ à C₁₄ ou (alkoxy en C₁ à C₄)-(alkyle en C₂ à C₆),
un reste cycloalkyle en C₃ à C₆ éventuellement substitué par un radical méthyle ou méthoxy, ou bien un reste phényle ou benzyle chacun éventuellement substitué par un radical fluoro, chloro, bromo, cyano, nitro, méthyle, méthoxy, trifluorométhyle ou trifluorométhoxy,
Q 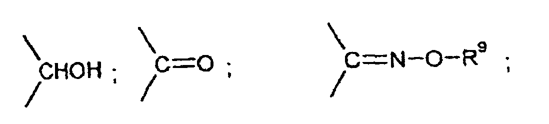 ou 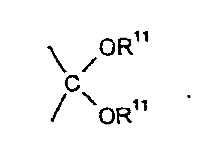
R⁹ représente l'hydrogène, un reste alkyle en C₁ à C₄, un reste cycloalkyle en C₃ à C₆ ou un reste phényle ou benzyle, chacun éventuellement substitué par un radical fluoro, chloro, bromo, méthyle, éthyle, n-propyle, isopropyle, tertiobutyle, méthoxy, trifluorométhyle, trifluorométhoxy, nitro ou cyano, ou bien un groupe CO-R¹' , CO₂R²', SO₂R¹' , CONH₂, CONHR¹¹ ou bien un groupe 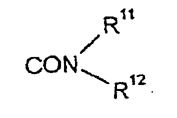
R¹¹ et R¹² sont identiques ou différents et représentent un reste méthyle ou éthyle,
m est égal à 1,
R^{1,} a, indépendamment de R¹, les définitions mentionnées ci-dessus de façon tout particulièrement appréciée pour R¹ et
R^{2,} a, indépendamment de R², les définitions mentionnées ci-dessus comme tout particulièrement apprécié pour R²_{.}

2. Procédé de production de composés de formule (I) suivant la revendication 1, **caractérisé en ce que,** pour obtenir des composés
(A) de formule (I-1-a) 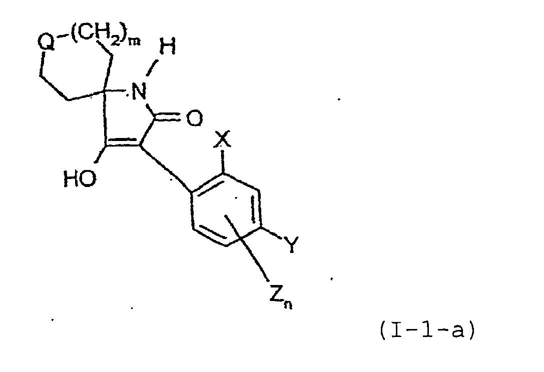 dans laquelle
Q, X, Y, Z, m et n ont les définitions indiquées dans la revendication 1,
on effectue la condensation intramoléculaire de composés de formule (II) 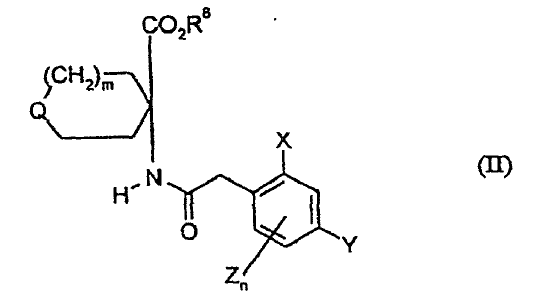 dans laquelle Q, X, Y, Z, m et n ont les définitions indiquées ci-dessus,
et
R⁸ représente un reste alkyle,
en présence d'un diluant et en présence d'une base,
et le cas échéant, on fait ensuite réagir les composés ainsi obtenus de formule (I-1-a), dans chaque cas
(Dα) avec des chlorures d'acides de formule (V) 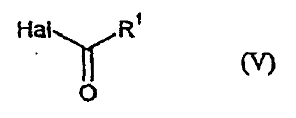 dans laquelle
R¹ a les définitions indiquées dans la revendication 1 et
Hal représente un halogène
ou bien
(β) avec des anhydrides d'acides dicarboxyliques de formule (VI)
R¹-CO-O-CO-R¹ (VI)
dans laquelle
R¹ a les définitions indiquées ci-dessus,
éventuellement en présence d'un diluant et, le cas échéant, en présence d'un accepteur d'acide ; ou bien
(E) on les fait réagir avec des esters d'acide chloroformique ou des thioesters d'acide chloroformique de formule (VII)
R²-M-CO-Cl (VII)
dans laquelle
R² et M ont les définitions indiquées dans la revendication 1,
éventuellement en présence d'un diluant et, le cas échéant, en présence d'un chlorure d'acide ; ou bien
(F) on les fait réagir avec des esters d'acide chloromonothioformique ou des esters d'acide chlorodithioformique de formule (VIII) 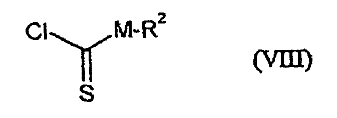 dans laquelle
M et R² ont les définitions indiquées ci-dessus, éventuellement en présence d'un diluant et, le cas échéant, en présence d'un accepteur d'acide; ou bien
(G) on les fait réagir avec des chlorures d'acides sulfoniques de formule (IX)
R³-SO₂-Cl (IX)
dans laquelle
R³ a la définition indiquée dans la revendication 1, éventuellement en présence d'un diluant et, le cas échéant, en présence d'un accepteur d'acide ; ou bien
(H) on les fait réagir avec des composés de phosphore de formule (X) 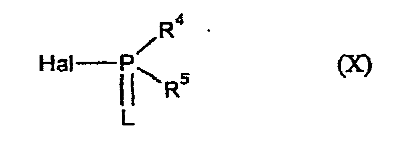 dans laquelle
L, R⁴ et R⁵ ont les définitions indiquées dans la revendication 1 et
Hal représente un halogène,
éventuellement en présence d'un diluant et, le cas échéant, en présence d'un accepteur d'acide ; ou bien
(I) on les fait réagir avec des composés métalliques ou des amines de formules (XI) ou (XII)
Me(OR¹³)ₜ (XI) 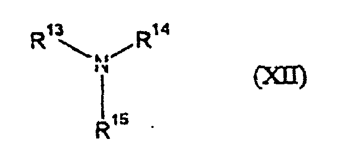
dans lesquelles
Me représente un métal monovalent ou divalent, t a la valeur 0 ou 1 et
R¹³, R¹⁴, R¹⁵ représentent, indépendamment les uns des autres l'hydrogène ou un reste alkyle, éventuellement en présence d'un diluant ; ou bien
(Jα) on les fait réagir avec des isocyanates ou des isothiocyanates de formule (XIII)
R⁶-N=C=L (XIII)
dans laquelle
R⁶ et L ont les définitions indiquées dans la revendication 1,
éventuellement en présence d'un diluant et, le cas échéant, en présence d'un catalyseur, ou bien
(β) on les fait réagir avec des chlorures d'acides carbamiques ou des chlorures d'acides thiocarbamiques de formule (XIV) 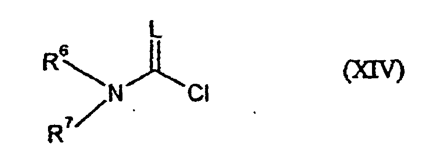 dans laquelle
L, R⁶ et R⁷ ont les définitions indiquées dans la revendication 1,
éventuellement en présence d'un diluant et, le cas échéant, en présence d'un accepteur d'acide.

3. Composés de formule (II) 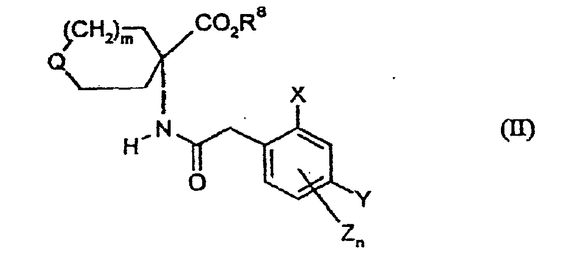 dans laquelle
Q, X, Y, Z, m et n ont les définitions indiquées ci-dessus et
R⁸ est un reste alkyle.

4. Composés de formule (XVII) 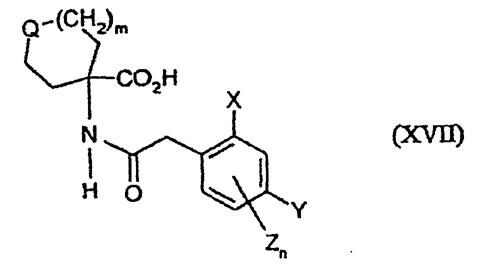 dans laquelle
Q, m, X, Y, Z et n ont les définitions indiquées dans la revendication 1.

5. Composés de formule (XX) 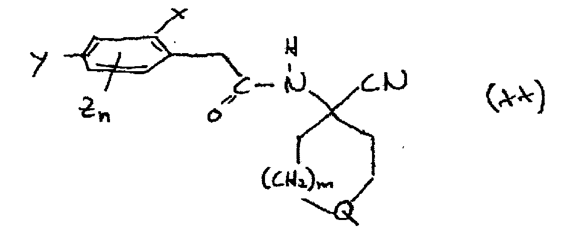 dans laquelle
X, Y, Z, n, m et Q ont la définition indiquée dans la revendication 1.

6. Compositions pesticides ou compositions herbicides **caractérisées par** une teneur en un composé de formule (I) suivant la revendication 1.

7. Utilisation de composés de formule (I) suivant la revendication 1 pour combattre des parasites et des mauvaises herbes.

8. Procédé de lutte contre des parasites et des mauvaises herbes, **caractérisé en ce qu'**on fait agir des composés de formule (I) suivant la revendication 1 sur les parasites ou les mauvaises herbes et/ou sur leur milieu.

9. Procédé de préparation de compositions pesticides et de compositions herbicides, **caractérisé en ce qu'**on mélange des composés de formule (I) suivant la revendication 1 avec des diluants et/ou des agents tensioactifs.
